(19) Europäisches Patentamt
European Patent Office
Office européen des brevets

(11) **EP 2 883 931 A1**

(12) **EUROPEAN PATENT APPLICATION**

(43) Date of publication:
**17.06.2015 Bulletin 2015/25**

(51) Int Cl.:
***C09K 8/68*** (2006.01)

(21) Application number: **13196827.3**

(22) Date of filing: **12.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Omya International AG**
**4665 Oftringen (CH)**

(72) Inventors:
• **Gantenbein, Daniel**
**6440 Elnesvagen (NO)**

• **Joachim Schoelkopf, Joachim**
**8956 Killwangen (CH)**
• **Gane, Patrick A. C.**
**4852 Rothrist (CH)**

(74) Representative: **Junge, Melanie**
**Maiwald Patentanwalts GmbH**
**Elisenhof**
**Elisenstraße 3**
**80335 München (DE)**

(54) **Improved gel stability**

(57) The present invention relates to a composition having a gel strength of from 1.0 µN to 10.0 N, a method of preparing a composition having a gel strength of from 1.0 µN to 10.0 N, a method of hydraulically fracturing of a subterranean formation, preferably an oil well or gas well, a hydraulically fracturing fluid as well as the use of said composition for hydraulic fracturing of a subterranean formation. The present invention further relates to the gel and use of this gel in pharmaceutical applications, cosmetic applications, constructing applications, paper applications, paint applications, plastic applications, food applications and/or agricultural applications.

Fig. 1

**Description**

The present invention relates to a composition having a gel strength of from 1.0 $\mu$N to 10.0 N, a method of preparing a composition having a gel strength of from 1.0 $\mu$N to 10.0 N, a method of hydraulically fracturing of a subterranean formation, preferably an oil well or gas well, a hydraulically fracturing fluid as well as the use of said composition for hydraulic fracturing of a subterranean formation. The present invention further relates to the gel and use of this gel in pharmaceutical applications, cosmetic applications, constructing applications, paper applications, paint applications, plastic applications, food applications and/or agricultural applications.

Hydraulic fracturing is a technology in the oil and gas production industry to increase the permeability of rock formations. Small cracks in the rock are formed by introducing liquid under high pressure. In order to maintain these cracks so called "proppants" are filled into these cracks. Typically, such proppants are in form of glass or ceramic beads having a particle size in the region of about 0.5 mm. Due to their size they tend to settle fast. Therefore, the hydraulic fracturing liquid is typically set up in such a way that it provides a rheology at which no settling of these proppants is observed. According to the industry's standard, the hydraulic fracturing liquid is based on a gel comprising a gel former and a gel cross-linker. Typically, the gel former is based on guar and/or cellulose derivatives, while the cross-linkers are borax or zirconium compounds.

Such hydraulic fracturing liquids are described in a number of documents. For instance, US 5,874,385 A refers to a fracturing fluid composition for use in fracturing underground formations, such as oil or gas wells is described. It includes at least one anhydrous aliphatic alcohol, a modified guar gum polymer, and as a complexor, a sodium borate salt.

GB 2 382 363 A refers to the use of a spacing fluid for separating a cement slurry from drilling fluid and other borehole fluids present in a well. The spacer comprises a fluid and particles of a loading agent. The loading agent has a particle size less than 5 microns. The loading agent may be magnesium oxide or titanium oxide.

US 1,190,112 refers to a solid dispersing and stabilizing agent and a method of making the same from a holocellulose source.

US 7,135,231 B1 refers to methods of making composite particles, as well as methods of using such particles as a proppant in oil and gas well hydraulic fracturing. The high strength composite particle comprises a series of incrementally applied resin micro layer coatings such that each of the microlayer partial coatings are interleaved with each other.

US 2003/0207768 A1 refers to a well treatment fluid composition comprising a carrier fluid and an amphoteric surfactant, and optionally a viscosifying agent and proppant, which is well suited for use in fracturing coal beds to stimulate methane production. The composition preferably is a foam that comprises a gas such as nitrogen or air.

US 2005/0211435 A1 refers to a high temperature blocking gel contains a blend of an aqueous fluid and carboxymethyl guar and a crosslinking agent.

US 2009/0270282 A1 describes that hydraulic fracturing of a subterranean hydrocarbon reservoir is carried out by using an aqueous wellbore fluid which is an aqueous solution of a surfactant which has the formula$(R_1-X)nZ$, where $R_1$ is an aliphatic group comprising a $C_{10}$-$C_{25}$ principal straight chain bonded at a terminal carbon atom thereof to X, and comprising at least one $C_1$-$C_6$ side chain. X is a charged head group, Z is a counterion, and n is an integer which ensures that the surfactant is charge neutral.

US 2010/0300693 A1 describes improved total recovery of oil, condensate and associated gas in a subterranean formation such that said hydrocarbons are released by a hydraulic fracturing process with a non-gel hydraulic fracturing fluid that comprises an enzyme surfactant fluid with at least one anionic surfactant thereby forming a non-gel hydraulic fracturing fluid enzyme surfactant composition which is injected at 1 to 3 percent of total fracturing fluid during fracturing.

The article of S. Kesavan et al. (Macromolecules 1992, 25, 2026-2032) describes that measurements have been conducted on the linear viscoelastic properties of guar and hydroxypropyl guar polymer solutions cross-linked with borate ions. Chemical equilibria involving boric acid, borate ions, and borate ions associated with cis-diol sites on the polysaccharide chains determine the number of cross-links.

The article of A. Tayal et al. (Macromolecules 1999, 32, 5567-5574) describes the enzymatic degradation of guar-borax gels.

The article of R. Barati et al. (Journal of Applied Polymer Science 2012, Volume 126, Issue 2,587-592) discloses that polyethylenimine-dextran sulfate polyelectrolyte complexes (PECs) were used to entrap two enzymes used to degrade polymer gels following hydraulic fracturing of oil wells to obtain delayed release and to protect the enzyme from harsh conditions.

The article of H.R. Jafry et al. (Ind. Eng. Chem. Res., 2011, 50 (6), 3259-3264) refers to a study providing an insight into the effect of incorporating nanoparticles on the rheology of fracturing fluids. Vapor grown carbon fibers (VGCFs) are first coated with silica and subsequently functionalized with octadecyltrichlorosilane groups. These coated and functionalized fibers are then added to a fracturing fluid gel. The rheology of the fracking fluids is affected if the nanoparticles interact with the polysaccharide or with the borate.

The article of S. Shah (SPE Production & Operations 2009, Volume 24, Number 3, 381-395) refers to correlations for the prediction of frictional pressure loss of fracturing slurries in straight tubing and CT. They are developed on the basis of full-scale slurry-flow tests with 1½-in. CT and slurries prepared with 35 lbm/1,000 gal of guar gel. The extensive experiments were conducted at the full-scale CT-flow test facility.

The article of T.N. Castro Dantas et al. (Journal of Dispersion Science and Technology 2005, Volume 26, Issue 1) describes that due to serious limitations of many polymeric gels, a new generation of fracturing gels based upon viscoelastic surfactants has been developed. This work describes the commonly used fracturing gels.

The article of M.M. Samuel et al. (SPE Drilling & Completion 1999, volume 14, number 4, 240-246) describes a surfactant-based polymer-free fluid, ClearFRAC™, that consists of a quaternary ammonium salt derived from a long-chain fatty acid is described. In brine, it builds fluid viscosity and viscoelasticity due to the formation of highly entangled worm-like micelles.

However, such hydraulic fracturing fluids typically comprise high amounts of guar and/or cellulose derivatives which may cause problems during the preparation of the hydraulic fracturing fluids, its storage, and its subsequent use. If such a composition comprising guar and/or cellulose derivatives in high amounts comes in contact with a suitable cross-linker, the composition may suffer from a severe viscosity increase in the composition prior to the intended end use, as high viscous compositions are difficult to pump.

Thus, there is still a need for compositions which address the foregoing technical problems described and especially allows for providing compositions having a sufficient rheology while maintaining a high anti-settling behaviour such that the composition is suitable for use as hydraulic fracturing fluid.

Accordingly, it is an objective of the present invention to provide a composition which is suitable as base composition for a hydraulic fracturing fluid. A further objective of the present invention is to provide a composition having a sufficient rheology. Another objective of the present invention is to provide a composition providing a sufficient, i.e. a high, anti-settling behaviour with regard to proppants. An even further objective of the present invention is to provide a composition comprising a low amount of a polysaccharide. Further objectives can be gathered from the following description of the invention.

The foregoing and other objectives are solved by the subject-matter as defined herein in claim 1. Advantageous embodiments of the inventive composition are defined in the corresponding sub-claims.

According to one aspect of the present application, a composition having a gel strength of from 1.0 $\mu$N to 10.0 N is provided, the composition comprising:

a) from 1.0 to 60.0 wt.-%, based on the total weight of the composition, of a particulate material comprising at least one calcium carbonate having a weight median particle size $d_{50}$ value of 0.01 to 50.0 $\mu$m;
b) from 0.01 to 10.0 wt.-%, based on the total weight of the composition, of at least one polysaccharide comprising glucose units and/or galactose units and/or mannose units;
c) from 0.01 to 4.0 wt.-%, based on the total weight of the composition, of at least one cross-linker suitable for cross-linking the at least one polysaccharide, and
d) at least 26.0 wt.-%, based on the total weight of the composition, of water.

The inventors surprisingly found out that the foregoing composition can be used in an hydraulic fracturing fluid. In particular, it has been found out that the composition having a gel strength of from 1.0 $\mu$N to 10.0 N according to the present invention provides a sufficient rheology in combination with a high anti-settling behaviour. Furthermore, the composition can be prepared by using a lower amount of a polysaccharide compared to the prior art at the same or higher gel strength.

It should be understood that for the purposes of the present invention, the following terms have the following meanings:

The term "calcium carbonate" according to the present invention refers to a material that comprises at least 80.0 wt.-%, preferably at least 85.0 wt.-%, more preferably at least 90.0 wt.-%, and most preferably at least 95.0 wt.-%, like at least 98.0 wt.-%, of calcium carbonate, based on the total dry weight of the calcium carbonate.

The term "polysaccharide comprising glucose units and/or galactose units and/or mannose units" in the meaning of the present invention refers to polymeric carbohydrate structures, formed by a plurality of glucose and/or galactose and/or mannose units joined together by glycosidic bonds. These structures are often linear, but may contain various degrees of branching.

As used herein and as generally defined in the art, the "$d_{50}$" value is determined based on measurements made by using a Sedigraph™ 5100 of Micromeritics Instrument Corporation (operating instrument software version 1.04) and is defined as the size at which 50 % (the median point) of the particle volume or mass is accounted for by particles having a diameter equal to the specified value. The method and the instrument are known to the skilled person and are commonly used to determine grain size of fillers and pigments. The measurement is carried out in an aqueous solution of 0.1 wt.-% $Na_4P_2O_7$. The samples are dispersed using a high speed stirrer and supersonics.

Where the term "comprising" is used in the present description and claims, it does not exclude other non-specified elements of major or minor functional importance. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Whenever the terms "including" or "having" are used, these terms are meant to be equivalent to "comprising" as defined above.

Where an indefinite or definite article is used when referring to a singular noun, e.g. "a", "an" or "the", this includes a plural of that noun unless something else is specifically stated.

According to another aspect of the present invention, a method of preparing a composition having a gel strength of from 1.0 $\mu$N to 10.0 N is provided, the method comprising at least the steps of:

a) providing a particulate material comprising at least one calcium carbonate in an amount of 1.0 to 60.0 wt.-%, based on the total weight of the composition;
b) providing at least one polysaccharide comprising glucose units and/or galactose units and/or mannose units in an amount of 0.01 to 10.0 wt.-%, based on the total weight of the composition;
c) providing at least one cross-linker in an amount of 0.01 to 4.0 wt.-%, based on the total weight of the composition;
d) optionally providing at least one gel delaying agent,
e) contacting the particulate material comprising at least one calcium carbonate of step a) with the at least one polysaccharide of step b), the at least one cross-linker of step c) and the optional at least one gel delaying agent of step d) to form the composition.

It is preferred that i) the particulate material comprising at least one calcium carbonate of step a) is provided in form of a powder or slurry, preferably a slurry having solids content of at least 5.0 wt.-%, preferably from 25.0 wt.-% to 85.0 wt.-%, more preferably from 35.0 wt.-% to 85.0 wt.-%, even more preferably from 40.0 wt.-% to 80.0 wt.-% and most preferably from 50.0 wt.-% to 80.0 wt.-%, based on the total weight of the slurry, and/or ii) the at least one polysaccharide of step b) is in form of a hydrocolloidal suspension or a dry material, preferably in form of a hydrocolloidal suspension having a polysaccharide concentration from 0.01 to 10.0 wt.-%, preferably from 0.01 to 3.5 wt.-%, more preferably from 0.05 to 3.0 wt.-% and most preferably from 0.1 to 2.5 wt.-%, based on the total weight of the suspension, and/or iii) the at least one cross-linker of step c) is in form of a solution or a dry material, preferably in form of a solution having a cross-linker concentration from 0.01 to 60.0 wt.-%, preferably from 1.0 to 50.0 wt.-%, more preferably from 10.0 to 50.0 wt.-% and most preferably from 20.0 to 50.0 wt.-%, based on the total weight of the solution, and/or iv) the optional at least one gel delaying agent of step d) is in form of a solution or a dry material, preferably in form of a dry material. It is further preferred that contacting step e) is carried out in that a) the particulate material of step a) is added to the at least one polysaccharide of step b) to form a mixture of the particulate material and the at least one polysaccharide, and b) the at least one cross-linker of step c) and the optional at least one gel delaying agent of step d) is/are added to the mixture of the particulate material and the at least one polysaccharide.

According to a further aspect of the present invention, a method of hydraulically fracturing of a subterranean formation, preferably an oil well or gas well, is provided, the method comprising at least the steps of:

a) providing a particulate material comprising at least one calcium carbonate having a weight median particle size $d_{50}$ value of 0.01 to 50.0 $\mu$m as defined herein,
b) providing at least one polysaccharide comprising glucose units and/or galactose units and/or mannose units as defined herein,
c) providing at least one cross-linker suitable for cross-linking the at least one polysaccharide of step b)
d) providing a proppant having a Mohs hardness of at least 2.5,
e) mixing the particulate material of step a) with the at least one polysaccharide of step b) for forming a mixture of the particulate material and the at least one polysaccharide,
f) mixing the mixture obtained in step e) with the at least one cross-linker of step c) and the proppant of step d) for forming a hydraulic fracturing fluid, and
g) injecting the hydraulic fracturing fluid formed in step f) into a subterranean formation, preferably an oil well or gas well, at a flow rate from 150 to 3001/s and a pressure rate from 500 to 1500 bar, for hydraulically fracturing the formation.

It is preferred that the proppant is selected from the group comprising sand, glass, ceramics, like lightweight ceramics, alumina, mica, corundum, bauxite, mullite, rutile, barite, calcium oxide and mixtures thereof. It is further preferred that the method further comprises step h) of contacting the hydraulic fracturing fluid in the subterranean formation with at least one gel breaking agent, preferably a gel breaking agent selected from enzymes, oxidants and mixtures thereof.

If the gel breaking agent is an oxidant, the gel breaking agent is selected from the group comprising ammonium perborate, sodium perborate, ammonium persulfate, sodium persulfate, calcium peroxide, magnesium oxide and mixtures thereof. Preferably, the gel breaking agent is selected from sodium perborate, ammonium persulfate and mixtures thereof.

Additionally or alternatively, if the gel breaking agent is an enzyme, the gel breaking agents can be a hydrolase, preferably a hydrolase that is stable and remains active at a pH in the range of 6 to 12. Preferably, the hydrolase is selected from glycoside hydrolase, galactomannan hydrolase and mixtures thereof.

According to an even further aspect of the present invention, a hydraulic fracturing fluid is provided, the hydraulic fracturing fluid comprising the composition as defined herein and a proppant having a Mohs hardness of at least 2.5, preferably the proppant is selected from the group comprising natural proppants, like mineral proppants, synthetic proppants, silica proppants, ceramic proppants, metallic proppants and mixtures thereof.

According to a still further aspect of the present invention, the use of a composition as defined herein for hydraulic fracturing of a subterranean formation, preferably an oil well or gas well, is provided. According to an even further aspect of the present invention, the use of a composition for the preparation of a hydraulic fracturing fluid, comprising the composition as defined herein and a proppant having a Mohs hardness of at least 2.5 is provided, preferably the proppant is selected from the group comprising sand, glass, ceramics, like lightweight ceramics, alumina, mica, corundum, bauxite, mullite, rutile, barite, calcium oxide and mixtures thereof. According to a still further aspect of the present invention, the use of a composition as defined herein in pharmaceutical applications, cosmetic applications, constructing applications, paper applications, paint applications, plastic applications, food applications and/or agricultural applications is provided.

According to one embodiment of the present invention, a) the at least one calcium carbonate of the particulate material is selected from among natural calcium carbonate (GCC), precipitated calcium carbonate (PCC), modified calcium carbonate (MCC) and mixtures thereof, and/or b) the particulate material further comprises a mineral material selected from mica, talc, dolomite, feldspar, bentonite, kaolinite, magnesite, muscovite, huntite and mixtures thereof.

According to another embodiment of the present invention, the at least one calcium carbonate of the particulate material has a) a weight median particle size $d_{50}$ from 0.01 to 25.0 $\mu$m, preferably from 0.1 to 20.0 $\mu$m, more preferably from 0.1 to 10.0 $\mu$m, and most preferably from 0.2 to 5.0 $\mu$m, and/or b) a specific surface area of from 0.1 to 200.0 m$^2$/g, more preferably 3.0 to 25.0 m$^2$/g, most preferably 5.0 to 15.0 m$^2$/g and even more preferably 6.0 to 12.0 m$^2$/g, measured using nitrogen and the BET method.

According to yet another embodiment of the present invention, the at least one polysaccharide comprises a) glucose units, preferably a linear chain of 1,4-linked $\beta$-D-glucopyranosyl units, more preferably the linear chain of 1,4-linked $\beta$-D-glucopyranosyl units is 1,6-linked with $\alpha$-D-glucopyranosyl units, or b) galactose and mannose units, preferably a linear chain of 1,4-linked $\beta$-D-mannopyranosyl units, more preferably the linear chain of 1,4-linked $\beta$-D-mannopyranosyl units is 1,6-linked with $\alpha$-D-galactopyranosyl units.

According to one embodiment of the present invention, the at least one polysaccharide comprising galactose and mannose units has a ratio of mannose units to galactose units from 6:1 1 to 1:1, preferably from 5:1 1 to 1:1, more preferably from 4:1 1 to 1:1 and most preferably from 3:1 1 to 1:1.

According to yet another embodiment of the present invention, a) the at least one polysaccharide is cellulose and/or a cellulose derivative and/or guar and/or a guar derivative, and/or b) the at least one polysaccharide is selected from the group comprising carboxymethyl cellulose, hydroxymethyl cellulose, hydroxyethyl cellulose, hydroxypropyl cellulose, hydroxyhexyl cellulose, guar gum, hydroxyethyl guar, hydroxypropyl guar, carboxymethyl guar, carboxymethyl hydroxyethyl guar, carboxymethyl hydroxypropyl guar, gum tragacanth, gum shiraz, gum ghatti, xanthan gum, scleroglucan gum, wellan gum, gelan gum and mixtures thereof.

According to one embodiment of the present invention, the at least one polysaccharide comprises galactose units and further units selected from arabinose units, rhamnose units, glucoronic acid units and mixtures thereof, preferably the at least one polysaccharide comprises galactose units, arabinose units, rhamnose units and glucoronic acid units.

According to another embodiment of the present invention, the at least one cross-linker is selected from the group comprising sodium tetraborate, a zirconium compound and mixtures thereof, preferably the at least one cross-linker is selected from the group comprising ammonium zirconium carbonate, potassium zirconium carbonate, zirconium lactate, zirconium glycolate, zirconium lactate triethanolamine, potassium pyroantimonate, potassium antimony tartrate, fused potassium antimony tartrate, antimony oxalate, antimony tartrate, and antimony ammonium fluoride and mixtures thereof.

According to yet another embodiment of the present invention, the composition further comprises at least one gel delaying agent, preferably a gel delaying agent selected from mono-, di- tri- or polyalcohols, alcohol amines, acids and mixtures thereof.

According to one embodiment of the present invention, the composition has a pH in the range of 5 to 12, preferably in the range of 6 to 11.

As set out above, the inventive composition having a gel strength of from 1.0 $\mu$N to 10.0 N comprises a particulate material, at least one polysaccharide, at least one cross-linker and water as set out in points a) to d). In the following, it is referred to further details of the present invention and especially the foregoing points of the inventive composition.

According to point a) of the present invention, the instant composition having a gel strength of from 1.0 $\mu$N to 10.0 N comprises from 1.0 to 60.0 wt.-%, based on the total weight of the composition, of a particulate material comprising at least one calcium carbonate having a weight median particle size $d_{50}$ value of 0.01 to 50.0 $\mu$m.

It is thus one requirement of the present invention that the composition comprises a particulate material comprising at least one calcium carbonate.

The at least one calcium carbonate in the meaning of the present invention refers to a material selected from among ground (or natural) calcium carbonate (GCC), a precipitated calcium carbonate (PCC), a modified calcium carbonate (MCC) and mixtures thereof.

GCC is understood to be a naturally occurring form of calcium carbonate, mined from sedimentary rocks such as limestone or chalk, or from metamorphic marble rocks and processed through a treatment such as grinding, screening and/or fractionizing in wet and/or dry form, for example by a cyclone or classifier. In one embodiment of the present invention, the GCC is selected from the group comprising marble, chalk, limestone and mixtures thereof.

By contrast, calcium carbonate of the PCC type include synthetic calcium carbonate products obtained by carbonation of a slurry of calcium hydroxide, commonly referred to in the art as a slurry of lime or milk of lime when derived from finely divided calcium oxide particles in water or by precipitation out of an ionic salt solution. PCC may be rhombohedral and/or scalenohedral and/or aragonitic; preferred synthetic calcium carbonate or precipitated calcium carbonate comprising aragonitic, vateritic or calcitic mineralogical crystal forms or mixtures thereof.

MCC in the meaning of the present invention may feature a natural ground or precipitated calcium carbonate with an internal structure modification or a surface-reaction product. According to one embodiment of the present invention, the modified calcium carbonate is a surface-reacted calcium carbonate.

In one embodiment of the present invention, the at least one calcium carbonate is selected from marble, chalk, limestone and mixtures thereof.

The expression "at least one calcium carbonate" means that one or more kinds of calcium carbonate may be present in the inventive composition.

Accordingly, it is appreciated that the at least one calcium carbonate may be a mixture of two or more kinds of calcium carbonate. For example, if the at least one calcium carbonate is a mixture of two or more calcium carbonates, one filler material can be marble, while the second or further calcium carbonate is selected from the group comprising chalk, limestone, PCC of aragonitic, vateritic or calcitic mineralogical crystal forms, MCC and mixtures thereof.

In one embodiment of the present invention, the at least one calcium carbonate is one kind of calcium carbonate. Preferably, the at least one calcium carbonate is marble or limestone.

It is one requirement of the present invention that the at least one calcium carbonate having a weight median particle size $d_{50}$ value of 0.01 to 50.0 $\mu$m. For example, the at least one calcium carbonate has a weight median particle size $d_{50}$ from 0.01 to 25.0 $\mu$m, preferably from 0.1 to 20.0 $\mu$m, more preferably from 0.1 to 10.0 $\mu$m, and most preferably from 0.2 to 5.0 $\mu$m.

Furthermore, the at least one calcium carbonate can have a top cut ($d_{98}$) of $\leq$ 150.0 $\mu$m. For example, the at least one calcium carbonate has a top cut ($d_{98}$) of $\leq$ 50.0 $\mu$m, preferably of $\leq$ 20.0 $\mu$m and most preferably of $\leq$ 7.5 $\mu$m

As used herein and as generally defined in the art, the "$d_{50}$" value is determined based on measurements made by using a Sedigraph™ 5100 of Micromeritics Instrument Corporation (operating instrument software version 1.04) and is defined as the size at which 50 % (the median point) of the particle volume or mass is accounted for by particles having a diameter equal to the specified value. The method and the instrument are known to the skilled person and are commonly used to determine grain size of fillers and pigments. The measurement is carried out in an aqueous solution of 0.1 wt.-% $Na_4P_2O_7$. The samples are dispersed using a high speed stirrer and supersonics.

Additionally or alternatively, the at least one calcium carbonate has a BET specific surface area (BET) in the range from 0.1 m$^2$/g to 200.0 m$^2$/g as measured by the BET nitrogen method according to ISO 9277. For example, the at least one calcium carbonate has a BET specific surface area (BET) in the range from 3.0 m$^2$/g to 25.0 m$^2$/g, more preferably 5.0 to 15.0 m$^2$/g and most preferably 6.0 to 12.0 m$^2$/g, measured using nitrogen and the BET method as measured by the BET nitrogen method according to ISO 9277.

In one embodiment of the present invention, the at least one calcium carbonate is preferably a marble or limestone having a weight median particle size diameter $d_{50}$ value from 0.01 $\mu$m to 50.0 $\mu$m, preferably from 0.01 $\mu$m to 25.0 $\mu$m, more preferably from 0.1 $\mu$m to 20.0 $\mu$m, even more preferably from 0.1 $\mu$m to 10.0 $\mu$m and most preferably from 0.2 $\mu$m to 5.0 $\mu$m. In this case, the at least one calcium carbonate exhibits a specific surface area (BET) in the range from 0.1 m$^2$/g to 200.0 m$^2$/g, preferably from 3.0 m$^2$/g to 25.0 m$^2$/g, more preferably from 5.0 to 15.0 m$^2$/g and most preferably from 6.0 to 12.0 m$^2$/g, measured using nitrogen and the BET method as measured by the BET nitrogen method according to ISO 9277.

It is preferred that the at least one calcium carbonate is a dry ground material, a material being wet ground and dried or a mixture of the foregoing materials. In general, the grinding step can be carried out with any conventional grinding device, for example, under conditions such that refinement predominantly results from impacts with a secondary body, i.e. in one or more of: a ball mill, a rod mill, a vibrating mill, a roll crusher, a centrifugal impact mill, a vertical bead mill an attrition mill, a pin mill, a hammer mill, a pulveriser, a shredder, a de-clumper, a knife cutter, or other such equipment known to the skilled man.

In case the at least one calcium carbonate is a wet ground calcium carbonate, the grinding step may be performed under conditions such that autogenous grinding takes place and/or by horizontal ball milling, and/or other such processes known to the skilled man. The wet processed ground calcium carbonate thus obtained may be washed and dewatered by well known processes, e.g. by flocculation, filtration or forced evaporation prior to drying. The subsequent step of drying may be carried out in a single step such as spray drying, or in at least two steps, e.g. by applying a first heating step to the calcium carbonate in order to reduce the associated moisture content to a level which is not greater than about 0.5 wt.-%, based on the total dry weight of the at least one calcium carbonate. The residual total moisture content of the filler can be measured by the Karl Fischer coulometric titration method, desorbing the moisture in an oven at 195 °C and passing it continuously into the KF coulometer (Mettler Toledo coulometric KF Titrator C30, combined with Mettler oven DO 0337) using dry $N_2$ at 100 ml/min for 10 min. The residual total moisture content can be determined with a calibration curve and also a blind of 10 min gas flow without a sample can be taken into account. The residual total moisture content may be further reduced by applying a second heating step to the at least one calcium carbonate. In case said drying is carried out by more than one drying steps, the first step may be carried out by heating in a hot current of air, while the second and further drying steps are preferably carried out by an indirect heating in which the atmosphere in the corresponding vessel comprises a surface treatment agent. It is also common that the at least one calcium carbonate is subjected to a beneficiation step (such as a flotation, bleaching or magnetic separation step) to remove impurities.

In one embodiment of the present invention, the at least one calcium carbonate comprises a dry ground calcium carbonate. In another embodiment of the present invention, the at least one calcium carbonate is a material being wet ground in a horizontal ball mill, and subsequently dried by using the well known process of spray drying.

For example, in case a wet ground and spray dried marble is used as the at least one calcium carbonate, the residual total moisture content of the at least one calcium carbonate is preferably of from 0.01 to 0.1 wt.-%, more preferably from 0.02 to 0.08 wt.-% and most preferably from 0.04 to 0.07 wt.-%, based on the total dry weight of the at least one calcium carbonate. If a PCC is used as the at least one calcium carbonate, the residual total moisture content of the calcium carbonate is preferably of from 0.01 to 0.2 wt.-%, more preferably from 0.05 to 0.17 wt.-% and most preferably from 0.05 to 0.10 wt.-%, based on the total dry weight of the at least one calcium carbonate.

It is appreciated that the term "particulate material" refers to a material comprising, preferably consisting of, at least one calcium carbonate. Accordingly, the term "particulate material" may refer to a material further comprising at least one material differing from the at least one calcium carbonate.

In one embodiment of the present invention, the particulate material consists of the at least one calcium carbonate.

Alternatively, the particulate material can further comprise a mineral material. For example, the particulate material further comprises a mineral material being selected from mica, talc, dolomite, feldspar, bentonite, kaolinite, magnesite, muscovite, huntite and mixtures thereof. Preferably, the particulate material further comprises a mineral material selected from mica, talc, dolomite and bentonite.

Thus, if the particulate material further comprises a mineral material selected from mica, talc, dolomite and bentonite, the particulate material consists of the at least one calcium carbonate and mica or calcium carbonate and talc or calcium carbonate and dolomite or calcium carbonate and bentonite. For example, the particulate material further comprises a mineral material selected from talc, dolomite and bentonite. Thus, if the particulate material further comprises a mineral material selected from talc, dolomite and bentonite, the particulate material consists of the at least one calcium carbonate and talc or calcium carbonate and dolomite or calcium carbonate and bentonite.

Preferably, the amount of the at least one calcium carbonate in the particulate material is at least 10.0 wt.-%, e.g. at least 20.0 wt.-%, preferably from 30.0 to 100.0 wt.-%, more preferably from 40.0 to 98.0 wt.-%, based on the total dry weight of the particulate material.

In one embodiment of the present invention, the weight median particle size $d_{50}$ value of the particulate material and of the at least one calcium carbonate is the same. Thus, it is appreciated that the particulate material has a weight median particle size $d_{50}$ value of 0.01 to 50.0 $\mu$m. For example, the particulate material has a weight median particle size $d_{50}$ from 0.01 to 25.0 $\mu$m, preferably from 0.1 to 20.0 $\mu$m, more preferably from 0.1 to 10.0 $\mu$m, and most preferably from 0.2 to 5.0 $\mu$m.

Accordingly, if the particulate material further comprises a mineral material selected from mica, talc, dolomite, feldspar, bentonite, kaolinite, magnesite, muscovite, huntite and mixtures thereof, the mineral material preferable has a weight median particle size $d_{50}$ value of 0.01 to 50.0 $\mu$m, preferably from 0.01 to 25.0 $\mu$m, more preferably from 0.1 to 20.0 $\mu$m, even more preferably from 0.1 to 10.0 $\mu$m and most preferably from 0.2 to 5.0 $\mu$m.

In accordance with the present invention, the composition having a gel strength of from 1.0 $\mu$N to 10.0 N comprises the particulate material in an amount of from 0.1 to 60.0 wt.-%, based on the total weight of the composition.

For example, the composition having a gel strength of from 1.0 μN to 10.0 N comprises the particulate material in an amount of from 1.0 to 50.0 wt.-%, preferably of from 5.0 to 40.0 wt.-% and most preferably of from 5.0 to 30.0 wt.-%, based on the total weight of the composition.

In one embodiment of the present invention, the instant composition can comprise a dispersing agent. Accordingly, it is appreciated that the particulate material comprising at least one calcium carbonate can be a dispersed particulate material. Dispersing agents suitable for dispersing such particulate materials are well known to the skilled person. For example, the dispersing agent can be selected from sodium polyacrylate, like sodium polyacrylate having a molecular weight $M_w$ of from 4 000 to 10 000 g/mol, preferably from 4 000 to 8 000 g/mol and most preferably of about 6 000 g/mol, sodium hydrogen phosphate and mixtures thereof. In one embodiment of the present invention, the dispersing agent is a mixture of sodium polyacrylate and sodium hydrogen phosphate.

If the instant composition comprises a dispersing agent, the dispersing agent is preferably present in a total amount of from 0.05 to 2.0 wt.-%, more preferably from 0.1 to 1.5 wt.-% and most preferably from 0.3 to 1.0 wt.-%, based on the total dry weight of the particulate material.

Alternatively, the instant composition is free of dispersing agents. Accordingly, it is appreciated that the particulate material is an undispersed particulate material.

It is a further requirement of the instant composition having a gel strength of from 1.0 μN to 10.0 N that it comprises from 0.01 to 10.0 wt.-%, based on the total weight of the composition, of at least one polysaccharide comprising glucose units and/or galactose units and/or mannose units.

It is appreciated that the expression "at least one" polysaccharide comprising glucose units and/or galactose units and/or mannose units means that one or more kinds of said polysaccharide may be present in the instant composition.

Accordingly, it should be noted that the polysaccharide comprising glucose units and/or galactose units and/or mannose units can be one kind of said polysaccharide. Alternatively, the at least one polysaccharide comprising glucose units and/or galactose units and/or mannose units can be a mixture of two or more kinds of said polysaccharide. For example, the at least one polysaccharide comprising glucose units and/or galactose units and/or mannose units can be a mixture of two or three kinds of said polysaccharide, like two kinds of said polysaccharide.

In one embodiment of the present invention, the at least one polysaccharide comprising glucose units and/or galactose units and/or mannose units is one kind of said polysaccharide.

According to the present invention, the at least one polysaccharide comprising glucose units and/or galactose units and/or mannose units is composed of a plurality of repeating monosaccharide units (at least 10) of glucose and/or galactose and/or mannose.

In case all the monosaccharide units in the at least one polysaccharide are of the same type, the polysaccharide is also named a homopolysaccharide or homoglycan. If the at least one polysaccharide is composed of more than one type of monosaccharide units, the polysaccharide is also named a heteropolysaccharide or heteroglycan.

Accordingly, it is appreciated that the at least one polysaccharide is a homopolysaccharide or a heteropolysaccharide.

In one embodiment of the present invention, the at least one polysaccharide is a homopolysaccharide. Preferably, the homopolysaccharide is composed of a plurality of repeating monosaccharide units of glucose. More preferably, the repeating monosaccharide units of glucose are α-D-glucoyranosyl units and/orβ -D-glucopyranosyl units. For example, the homopolysaccharide is composed of repeating units of α-D-glucoyranosyl units or β-D-glucopyranosyl units. In one embodiment of the present invention, the homopolysaccharide is a linear chain of 1,4-linked β-D-glucopyranosyl units.

Additionally or alternatively, the homopolysaccharide comprises α -D-glucopyranosyl units which are 1,6-linked to the linear chain of 1,4-linked β -D-glucopyranosyl units.

In one embodiment of the present invention, the at least one polysaccharide is a homopolysaccharide being composed of a plurality of repeating monosaccharide units of galactose. Preferably, the repeating monosaccharide units of galactose are α-D-galactopyranosyl units and/or β-D-galactopyranosyl units. In one embodiment of the present invention, the homopolysaccharide is composed of repeating units of β-D-galactopyranose or α-D-galactopyranose. For example, the homopolysaccharide is a linear chain of 1,4-linked β-D-galactopyranosyl units.

In one embodiment of the present invention, the at least one polysaccharide is a homopolysaccharide being composed of a plurality of repeating monosaccharide units of mannose. Preferably, the repeating monosaccharide units of mannose are α-D-mannopyranosyl units and/or β-D-mannopyranosyl units. In one embodiment of the present invention, the homopolysaccharide is composed of repeating units of β-D-mannopyranose or α-D-mannopyranose. For example, the homopolysaccharide is a linear chain of 1,4-linked β-D-mannopyranosyl units.

In another embodiment of the present invention, the at least one polysaccharide is a heteropolysaccharide comprising mannose units and galactose units. Preferably, the heteropolysaccharide comprises galactose units selected from α-D-galactopyranose, β-D-galactopyranose and mixtures thereof and mannose units selected from α-D-mannopyranose, β-D-mannopyranose and mixtures thereof.

For example, the heteropolysaccharide comprises β-D-mannopyranosyl units and α-D-galactopyranosyl units.

In one embodiment of the present invention, the heteropolysaccharide comprises a linear chain of 1,4-linked β-D-mannopyranosyl units to which α-D-galactopyranosyl units are 1,6-linked. Preferably, the heteropolysaccharide comprises a linear chain of 1,4-linked β-D-mannopyranosyl units to which single α-D-galactopyranosyl units are 1,6-linked.

For example, the heteropolysaccharide comprises a linear chain of 1,4-linked β-D-mannopyranosyl units with 1,6-linked α-D-galactopyranosyl units on average to every second mannose unit.

If the at least one polysaccharide comprises a heteropolysaccharide comprising mannose units and galactose units, the at least one polysaccharide has a ratio of mannose units to galactose units from 6:1 1 to 1:1, preferably from 5:1 1 to 1:1, more preferably from 4:1 to 1:1 and most preferably from 3:1 to 1:1, e.g. in a ratio of mannose units to galactose units of 2:1.

Additionally or alternatively, a derivative of the at least one polysaccharide comprising glucose units and/or galactose units and/or mannose units may be utilized in the process of the present invention. Such derivative may be obtained, for example, by modifying the polysaccharide through the use of enzymes, acids, oxidation media, temperature, radiation etc. Methods for preparing such derivatives are known to the skilled person. For example, a modification may be obtained by etherification of the polysaccharide with propyleneoxide or ethyleneoxide resulting in a hydroxypropyl derivative or hydroxyethyl derivative.

In one embodiment of the present invention, the derivative of the at least one polysaccharide comprising glucose units and/or galactose units and/or mannose units is an anionic derivative of the at least one polysaccharide. For example, the anionic derivative of the at least one polysaccharide is a carboxymethyl derivative and/or carboxymethyl hydroxypropyl derivative and/or carboxymethyl hydroxyethyl derivative of the at least one polysaccharide.

In another embodiment of the present invention, the derivative of the at least one polysaccharide comprising glucose units and/or galactose units and/or mannose units is a cationic derivative of the at least one polysaccharide. For example, the cationic derivative of the at least one polysaccharide is obtained by reacting the polysaccharide with derivatives of quaternary ammonium salts.

Methods for preparing such anionic and/or cationic derivatives of the at least one polysaccharide comprising glucose units and/or galactose units and/or mannose units are known to the skilled person.

The cationic derivative and/or anionic derivative of the at least one polysaccharide comprising glucose units and/or galactose units and/or mannose units preferably has a degree of substitution of the hydroxyl groups of at least 0.01, more preferably of at least 0.05 and may be as high as 1.0. A suitable degree of substitution of the hydroxyl groups may be from 0.1 to 0.5.

The molecular weight of the at least one polysaccharide comprising glucose units and/or galactose units and/or mannose units preferably ranges from 1 000 to 1 000 000 Da and is generally about 220 000 Da. The molecular weight of the at least one polysaccharide comprising glucose units and/or galactose units and/or mannose units can be adjusted by the treatment with hydrogen peroxide ($H_2O_2$).

In one embodiment of the present invention, the at least one polysaccharide is cellulose and/or a cellulose derivative and/or guar and/or a guar derivative. For example, the at least one polysaccharide is cellulose or a cellulose derivative or guar or a guar derivative. Preferably, the at least one polysaccharide is cellulose or guar, like guar.

In another embodiment of the present invention, the at least one polysaccharide is a cellulose derivative or a guar derivative, like a guar derivative. For example, the at least one polysaccharide is a cellulose derivative selected from carboxymethyl cellulose, hydroxymethyl cellulose, hydroxyethyl cellulose, hydroxypropyl cellulose, hydroxyhexyl cellulose and mixtures thereof. Alternatively, the at least one polysaccharide is a guar derivative selected from guar gum, hydroxyethyl guar, hydroxypropyl guar, carboxymethyl guar, carboxymethyl hydroxyethyl guar, carboxymethyl hydroxypropyl guar and mixtures thereof.

In one embodiment of the present invention, the at least one polysaccharide is selected from the group comprising gum tragacanth, gum shiraz, gum ghatti, xanthan gum, scleroglucan gum, wellan gum, gelan gum and mixtures thereof.

Accordingly, the at least one polysaccharide may further comprise monosaccharide units differing from glucose units and/or galactose units and/or mannose units. If the at least one polysaccharide further comprises monosaccharide units differing from glucose units and/or galactose units and/or mannose units, the amount of glucose units and/or galactose units and/or mannose units is ≥ 50.0 wt.-%, preferably ≥ 60.0 wt.-%, more preferably ≥ 70.0 wt.-% and most preferably ≥ 80.0 wt.-%, based on the total weight of the at least one polysaccharide.

If the at least one polysaccharide further comprises monosaccharide units differing from glucose units and/or galactose units and/or mannose units, the at least one polysaccharide preferably comprises galactose units and further units selected from arabinose units, rhamnose units, glucoronic acid units and mixtures thereof. For example, the at least one polysaccharide comprises galactose units, arabinose units, rhamnose units and glucoronic acid units.

In accordance with the present invention, the composition having a gel strength of from 1.0 μN to 10.0 N comprises the at least one polysaccharide in an amount of from 0.01 to 10.0 wt.-%, based on the total weight of the composition. For example, the composition having a gel strength of from 1.0 μN to 10.0 N comprises the at least one polysaccharide in an amount of from 0.05 to 9.0 wt.-%, preferably of from 0.1 to 7.5 wt.-% and most preferably of from 0.15 to 5.0 wt.-%, based on the total weight of the composition.

It is appreciated that instant composition having a gel strength of from 1.0 $\mu$N to 10.0 comprises the at least one polysaccharide comprising glucose units and/or galactose units and/or mannose units in the form of the corresponding cross-linked polysaccharide. The cross-linked polysaccharide is preferably obtained by contacting the at least one polysaccharide comprising glucose units and/or galactose units and/or mannose units with at least one cross-linker suitable for cross-linking the at least one polysaccharide.

Thus, it is one further requirement of the instant invention that the composition having a gel strength of from 1.0 $\mu$N to 10.0 N comprises from 0.01 to 4.0 wt.-%, based on the total weight of the composition, of at least one cross-linker suitable for cross-linking the at least one polysaccharide.

It is appreciated that the expression "at least one" cross-linker suitable for crosslinking the at least one polysaccharide means that one or more kinds of cross-linker can be present in the instant composition.

Accordingly, it should be noted that the at least one cross-linker suitable for crosslinking the at least one polysaccharide can be one kind of cross-linker. Alternatively, the at least one cross-linker suitable for cross-linking the at least one polysaccharide can be a mixture of two or more kinds of cross-linker. For example, the at least one cross-linker suitable for cross-linking the at least one polysaccharide can be a mixture of two or three kinds of cross-linker, like two kinds of cross-linker.

In one embodiment of the present invention, the at least one cross-linker suitable for cross-linking the at least one polysaccharide is one kind of cross-linker.

It is appreciated that at least one cross-linker suitable for cross-linking the at least one polysaccharide can be any cross-linker that is suitable for cross-linking the at least one polysaccharide.

In one embodiment of the present invention, the at least one cross-linker suitable for cross-linking the at least one polysaccharide is at least one cross-linker selected from the group comprising sodium tetraborate, a zirconium compound and mixtures thereof. For example, the at least one cross-linker suitable for cross-linking the at least one polysaccharide is at least one cross-linker selected from a zirconium compound.

In one embodiment of the present invention, at least one cross-linker suitable for cross-linking the at least one polysaccharide is selected from the group comprising ammonium zirconium carbonate, potassium zirconium carbonate, zirconium lactate, zirconium glycolate, zirconium lactate triethanolamine, potassium pyroantimonate, potassium antimony tartrate, fused potassium antimony tartrate, antimony oxalate, antimony tartrate, and antimony ammonium fluoride and mixtures thereof.

In accordance with the present invention, the composition having a gel strength of from 1.0 $\mu$N to 10.0 N comprises the at least one cross-linker suitable for crosslinking the at least one polysaccharide in an amount of from 0.01 to 4.0 wt.-% wt.-%, based on the total weight of the composition. For example, the composition having a gel strength of from 1.0 $\mu$N to 10.0 N comprises the at least one cross-linker suitable for cross-linking the at least one polysaccharide in an amount of from 0.01 to 3.5 wt.-%, preferably of from 0.05 to 3.0 wt.-% and most preferably of from 0.1 to 2.5 wt.-%, based on the total weight of the composition.

According to another requirement of the present invention, the instant composition comprises at least 26.0 wt.-%, based on the total weight of the composition, of water. For example, the instant composition comprises from 26.0 to 98.98 wt.-% of water, based on the total weight of the composition.

It is appreciated that the remaining part to 100.0 wt.-% based on the total weight of the composition, refers to the water present in the composition.

For example, the instant composition comprises, preferably consists of:

a) from 1.0 to 50.0 wt.-%, based on the total weight of the composition, of a particulate material comprising at least one calcium carbonate having a weight median particle size $d_{50}$ value of 0.01 to 50.0 $\mu$m;
b) from 0.05 to 9.0 wt.-%, based on the total weight of the composition, of at least one polysaccharide comprising glucose units and/or galactose units and/or mannose units;
c) from 0.01 to 3.5 wt.-%, based on the total weight of the composition, of at least one cross-linker suitable for cross-linking the at least one polysaccharide, and
d) at least 37.5 wt.-%, preferably from 37.5 to 98.94 wt.-%, based on the total weight of the composition, of water.

In one embodiment of the present invention, the instant composition comprises, preferably consists of:

a) from 5.0 to 40.0 wt.-%, based on the total weight of the composition, of a particulate material comprising at least one calcium carbonate having a weight median particle size $d_{50}$ value of 0.01 to 50.0 $\mu$m;
b) from 0.1 to 7.5 wt.-%, based on the total weight of the composition, of at least one polysaccharide comprising glucose units and/or galactose units and/or mannose units;
c) from 0.05 to 3.0 wt.-%, based on the total weight of the composition, of at least one cross-linker suitable for cross-linking the at least one polysaccharide, and

d) at least 49.5 wt.-%, preferably from 49.5 to 94.85 wt.-%, based on the total weight of the composition, of water.

In one embodiment of the present invention, the instant composition comprises, preferably consists of

a) from 5.0 to 30.0 wt.-%, based on the total weight of the composition, of a particulate material comprising at least one calcium carbonate having a weight median particle size $d_{50}$ value of 0.01 to 50.0 $\mu$m;
b) from 0.5 to 5.0 wt.-%, based on the total weight of the composition, of at least one polysaccharide comprising glucose units and/or galactose units and/or mannose units;
c) from 0.1 to 2.5 wt.-%, based on the total weight of the composition, of at least one cross-linker suitable for cross-linking the at least one polysaccharide, and
d) at least 62.5 wt.-%, preferably from 62.5 to 94.4 wt.-%, based on the total weight of the composition, of water.

It is appreciated that the water is industrial water, process water tap water and/or deionized water, preferably tap water or deionized water. For example, the water is tap water.

In one embodiment of the present invention, the water can comprise other solvents, preferably organic solvents such as alcohols, like ethanol, or petroleum distillates.

For example, the water comprises other solvents, like organic solvents, in an amount of $\leq$ 30.0 wt.-%, preferably $\leq$ 20.0 wt.-% and most preferably $\leq$ 10.0 wt.-%, like from 1.0 to 10.0 wt.-%, based on the total weight of the water.

It is appreciated that the pH of the instant composition can vary in a broad range and can be adapted to the specific need. For example, the pH of the instant composition can be adapted to the pH of the soil to be treated with the instant composition.

In one embodiment of the present invention, the composition having a gel strength of from 1.0 $\mu$N to 10.0 N has a pH in the range of 6 to 12, preferably in the range of 7 to 11.

Additionally, the instant composition can further comprise at least one gel delaying agent. It is appreciated that the gel delaying agent can be any agent that is suitable for delaying the formation of a gel in the instant composition. For example, the at least one gel delaying agent can be selected from mono-, di- tri- or polyalcohols, such as methanol, ethanol, propanol, butanol, isopropanol, glycol, glycerol or sucrose, alcohol amines, such as mono-ethanlolamine, di-ethanol-amine or triethanolamine, acids such citric acid, or formic acid and mixtures thereof.

If the instant composition further comprises at least one gel delaying agent, the composition comprises the at least one gel delaying agent in an amount of from 0.01 to 10.0 wt.-%, preferably of from 0.5 to 8.0 wt.-% and most preferably of from 1.0 to 6.0 wt.-%, based on the total weight of the composition.

Optionally, the composition comprises further additives such as biocides, breakers, corrosion inhibitors, friction reducers, potassium chloride, oxygen scavengers, pH adjusting agents, scale inhibitors, surfactants and mixtures thereof. These further additives, if present, are typically present in the composition in a total amount from 0.01 to 2.0 wt.-%, based on the total weight of the composition.

Furthermore, it is appreciated that the instant composition has specific characteristics.

In particular, it is one requirement of the present invention that the composition has a gel strength of from 1.0 $\mu$N to 10.0 N. For example, the composition features a gel strength of from 10.0 $\mu$N to 5.0 N, preferably of from 100.0 $\mu$N to 1.0 N and most preferably of from 0.01 N to 0.5 N.

Regarding the gel strength, it is to be noted that the composition preferably features a higher gel strength than the same composition excluding a particulate material comprising at least one calcium carbonate having a weight median particle size $d_{50}$ value of 0.01 to 50.0 $\mu$m. That is to say, the inventive composition preferably features a higher gel strength if the composition comprises a particulate material comprising at least one calcium carbonate having a weight median particle size $d_{50}$ value of 0.01 to 50.0 $\mu$m.

Additionally or alternatively, it is to be noted that the instant composition comprises a lower amount of a polysaccharide at the same or higher gel strength compared to the same composition excluding a particulate material comprising at least one calcium carbonate having a weight median particle size $d_{50}$ value of 0.01 to 50.0 $\mu$m. That is to say, the inventive composition preferably comprises a lower amount of polysaccharide but features the same or higher gel strength if the composition comprises a particulate material comprising at least one calcium carbonate having a weight median particle size $d_{50}$ value of 0.01 to 50.0 $\mu$m.

In one embodiment of the present invention, the instant composition having a gel strength of from 1.0 $\mu$N to 10.0 N has been obtained by a process as described in the following.

According to another aspect of the present invention, a method of preparing a composition having a gel strength of from 1.0 $\mu$N to 10.0 N is provided, the method comprises at least the steps of:

a) providing a particulate material comprising at least one calcium carbonate in an amount of 1.0 to 60.0 wt.-%, based on the total weight of the composition;
b) providing at least one polysaccharide comprising glucose units and/or galactose units and/or mannose units in an amount of 0.01 to 10.0 wt.-%, based on the total weight of the composition;

c) providing at least one cross-linker in an amount of 0.01 to 4.0 wt.-%, based on the total weight of the composition;

d) optionally providing at least one gel delaying agent,

e) contacting the particulate material comprising at least one calcium carbonate of step a) with the at least one polysaccharide of step b), the at least one cross-linker of step c) and the optional at least one gel delaying agent of step d) to form the composition.

With regard to the composition having a gel strength of from 1.0 $\mu$N to 10.0 N as prepared by the method, the particulate material provided in step a), the at least one polysaccharide provided in step b), the at least one cross-linker provided in step c) and the at least one gel delaying agent optionally provided in step d), it is referred to the definitions set out above when defining the composition, the particulate material, the at least one polysaccharide, the at least one cross-linker and the optional at least one gel delaying agent.

It is one requirement of the present invention that the particulate material comprising at least one calcium carbonate is provided in an amount of 1.0 to 60.0 wt.-%, based on the total weight of the composition. For example, the particulate material is provided in an amount of from 1.0 to 50.0 wt.-%, preferably of from 5.0 to 40.0 wt.-% and most preferably of from 5.0 to 30.0 wt.-%, based on the total weight of the composition.

It is appreciated that the particulate material comprising at least one calcium carbonate is provided in form of a powder or slurry. Preferably, the particulate material comprising at least one calcium carbonate is provided in form of a slurry.

The term "slurry" in the meaning of the present invention comprises insoluble solids and water and optionally further additives and usually contains large amounts of solids and, thus, is more viscous and generally of higher density than the liquid from which it is formed.

If the particulate material is provided in form of a slurry, the slurry has solids content of at least 5.0 wt.-%, based on the total weight of the slurry. For example, the slurry has solids content of from 25.0 wt.-% to 85.0 wt.-%, more preferably from 35.0 wt.-% to 85.0 wt.-%, even more preferably from 40.0 wt.-% to 80.0 wt.-% and most preferably from 50.0 wt.-% to 80.0 wt.-%, based on the total weight of the slurry.

It is preferred that the slurry is an aqueous slurry.

It is a further requirement of the present invention that the at least one polysaccharide comprising glucose units and/or galactose units and/or mannose units is provided in an amount of 0.01 to 10.0 wt.-%, based on the total weight of the composition. For example, the at least one polysaccharide is provided in an amount of 0.05 to 9.0 wt.-%, preferably of from 0.1 to 7.5 wt.-% and most preferably of from 0.5 to 5.0 wt.-%, based on the total weight of the composition.

It is appreciated that the at least one polysaccharide comprising glucose units and/or galactose units and/or mannose units is provided in form of a hydrocolloidal suspension or a dry material. Preferably, the at least one polysaccharide comprising glucose units and/or galactose units and/or mannose units is provided in form of a hydrocolloidal suspension. It is appreciated that the use of a hydrocolloidal suspension of the at least one polysaccharide comprising glucose units and/or galactose units and/or mannose units is advantageous because it provides a faster and more complete dissolution as well as a more homogeneous distribution of the at least one polysaccharide in the instant composition.

The term "dry material" in the meaning of the present invention refers to material being in the form of a powder, i.e. the material has a moisture content of $\leq$ 0.5 wt.-%, preferably of $\leq$ 0.2 wt.-% and most preferably of $\leq$ 0.05 wt.-%, based on the total weight of the material.

The term "hydrocolloidal suspension" in the meaning of the present invention refers to a system comprising solvent and at least one polysaccharide, wherein the particles of the at least one polysaccharide comprising glucose units and/or galactose units and/or mannose units are dissolved in the solvent. The term "dissolved" in the meaning of the present invention refers to systems in which practically no discrete solid particles of the at least one polysaccharide are observed in the solvent.

If the at least one polysaccharide comprising glucose units and/or galactose units and/or mannose units is provided in form of a hydrocolloidal suspension, the hydrocolloidal suspension has preferably a polysaccharide concentration from 0.01 to 4.0 wt.-%, based on the total weight of the hydrocolloidal suspension. For example, the hydrocolloidal suspension has a polysaccharide concentration from 0.01 to 3.5 wt.-%, more preferably from 0.05 to 3.0 wt.-% and most preferably from 0.1 to 2.5 wt.-%, based on the total weight of the suspension.

Additionally or alternatively, the at least one cross-linker, which is provided in the instant composition in an amount of from 0.01 to 4.0 wt.-% wt.-%, preferably of from 0.01 to 3.5 wt.-%, more preferably of from 0.05 to 3.0 wt.-% and most preferably of from 0.1 to 2.5 wt.-%, based on the total weight of the composition, is in form of a solution or a dry material. Preferably, the at least one cross-linker, is provided in form of a solution. It is appreciated that the use of a solution of the at least one cross-linker is advantageous because it provides a more homogenous distribution in the instant composition and a possibility of controlling the method of hydraulically fracturing of a subterranean formation.

The term "solution" in the meaning of the present invention refers to a system comprising solvent and the at least one cross-linker, wherein the particles of the at least one cross-linker are dissolved in the solvent. The term "dissolved" in the meaning of the present invention refers to systems in which no discrete solid particles are observed in the solvent.

If the at least one cross-linker is provided in form of a solution, the solution has a cross-linker concentration from 0.01 to 60.0 wt.-%, based on the total weight of the solution. For example, the cross-linker solution has a cross-linker concentration from 1.0 to 50.0 wt.-%, more preferably from 10.0 to 50.0 wt.-% and most preferably from 20.0 to 50.0 wt.-%, based on the total weight of the solution.

It is preferred that the cross-linker solution is an aqueous cross-linker solution.

If the instant composition comprises at least one gel delaying agent, the at least one gel delaying agent is preferably provided in an amount of from 0.01 to 10.0 wt.-%, preferably of from 0.5 to 8.0 wt.-% and most preferably of from 1.0 to 6.0 wt.-%, based on the total weight of the composition.

It is appreciated that the at least one gel delaying agent is provided in form of a solution or a dry material. In one embodiment of the instant invention, the at least one gel delaying agent is provided in form of a solution. It is appreciated that the use of a solution of the at least one gel delaying agent is advantageous because it provides a faster and more complete dissolution as well as a more homogeneous distribution of the at least one gel delaying agent in the instant composition.

In one embodiment of the present invention, at least one of the mandatory components of the instant composition, i.e. the particulate material comprising at least one calcium carbonate, the at least one polysaccharide comprising glucose units and/or galactose units and/or mannose units and the at least one cross-linker, is provided in combination with water. That is to say, the particulate material is provided in form of a slurry and/or the at least one polysaccharide is provided in form of a hydrocolloidal suspension and/or the at least one cross-linker is provided in form of a solution.

In one embodiment of the present invention, the particulate material is provided in form of a slurry and the at least one polysaccharide is provided in form of a hydrocolloidal suspension and/or the at least one cross-linker is provided in form of a solution. For example, the particulate material is provided in form of a slurry and the at least one polysaccharide is provided in form of a hydrocolloidal suspension and the at least one cross-linker is provided in form of a solution.

Alternatively, the particulate material is provided in form of a slurry or the at least one polysaccharide is provided in form of a hydrocolloidal suspension and/or the at least one cross-linker is provided in form of a solution. For example, the particulate material is provided in form of a powder, the at least one polysaccharide is provided in form of a hydrocolloidal suspension and the at least one cross-linker is provided in form of a solution.

Accordingly, it is appreciated that the water present in the instant composition is preferably derived from the particulate material being provided in form of a slurry and/or the at least one polysaccharide being provided in form of a hydrocolloidal suspension and/or the at least one cross-linker being provided in form of a solution.

The step of contacting the particulate material of step a) with the at least one polysaccharide of step b), the at least one cross-linker of step c) and the optional at least one gel delaying agent of step d) to form the instant composition preferably takes place under mixing conditions. The skilled man will adapt these mixing conditions (such as the configuration of mixing pallets and mixing speed) according to his process equipment.

In one embodiment of the present invention, contacting step e) is carried out in that the particulate material of step a) is added to the at least one polysaccharide of step b) to form a mixture of the particulate material and the at least one polysaccharide.

The step of adding the particulate material of step a) to the at least one polysaccharide of step b) is preferably carried out in one or more steps. It is appreciated that this preferably takes place under mixing conditions. The skilled man will adapt these mixing conditions (such as the configuration of mixing pallets and mixing speed) according to his process equipment.

For example, it is possible to add the particulate material of step a) to the at least one polysaccharide of step b) in one portion. In one embodiment of the present invention, the particulate material of step a) is added to the at least one polysaccharide of step b) in more than one portion, wherein said particulate material is preferably added in about equal portions. Alternatively, it is also possible to add the particulate material of step a) in unequal portions to the at least one polysaccharide of step b), i.e. in larger and smaller portions.

In one embodiment of the present invention, the particulate material of step a) can be added to the at least one polysaccharide of step b) in a broad temperature range. That is to say, the particulate material of step a) can be added to the at least one polysaccharide of step b) at a temperature of ≤ 100 °C. For example, the particulate material of step a) can be added to the at least one polysaccharide of step b) at a temperature of from 5 to 100 °C, preferably of from 5 to 80 °C, more preferably of from 10 to 60 °C and most preferably of from 15 to 40 °C, like of about room temperature. A temperature of ≤ 100 °C is preferably adjusted if the particulate material of step a) is provided as slurry and/or the at least one polysaccharide of step b) is provided as hydrocolloidal suspension.

Additionally, contacting step e) is preferably carried out in that the at least one cross-linker of step c) and the optional at least one gel delaying agent of step d) is/are added to the mixture of the particulate material and the at least one polysaccharide.

The step of adding the at least one cross-linker of step c) and the optional at least one gel delaying agent of step d) to the mixture of the particulate material and the at least one polysaccharide is preferably carried out in one or more steps. It is appreciated that this preferably takes place under mixing conditions. The skilled man will adapt these mixing conditions (such as the configuration of mixing pallets and mixing speed) according to his process equipment.

For example, it is possible to add the at least one cross-linker of step c) and the optional at least one gel delaying agent of step d) to the mixture of the particulate material and the at least one polysaccharide in one portion. In one embodiment of the present invention, the at least one cross-linker of step c) and the optional at least one gel delaying agent of step d) is/are added to the mixture of the particulate material and the at least one polysaccharide in more than one portion, wherein said at least one cross-linker and the optional at least one gel delaying agent is/are preferably added in about equal portions. Alternatively, it is also possible to add the at least one cross-linker of step c) and the optional at least one gel delaying agent of step d) in unequal portions to the mixture of the particulate material and the at least one polysaccharide, i.e. in larger and smaller portions.

If the at least one gel delaying agent is provided in the instant method, the at least one cross-linker of step c) and the at least one gel delaying agent of step d) can be added separately or together to the mixture of the particulate material and the at least one polysaccharide.

In one embodiment of the present invention, the at least one cross-linker of step c) and the at least one gel delaying agent of step d) are added together to the mixture of the particulate material and the at least one polysaccharide. In this case, the at least one cross-linker of step c) and the at least one gel delaying agent of step d) are preferably provided in form of a solution. For example, the at least one gel delaying agent of step d) provided in form of a powder is added to the at least one cross-linker of step c) provided in form of a solution.

If the at least one cross-linker of step c) and the at least one gel delaying agent of step d) are added separately to the mixture of the particulate material and the at least one polysaccharide, the at least one cross-linker of step c) can be added before and/or after the at least one gel delaying agent of step d) to the mixture of the particulate material and the at least one polysaccharide. For example, the at least one cross-linker of step c) is added before the at least one gel delaying agent of step d) to the mixture of the particulate material and the at least one polysaccharide. In this case, the at least one cross-linker of step c) is preferably provided in form of a solution and the at least one gel delaying agent of step d) is preferably provided in form of a powder.

It is appreciated that the temperature adjusted for the addition of the at least one cross-linker of step c) and the optional at least one gel delaying agent of step d) to the mixture of the particulate material and the at least one polysaccharide is similar to the temperature adjusted during the addition of the particulate material of step a) to the at least one polysaccharide of step b). Thus, the at least one cross-linker of step c) and the optional at least one gel delaying agent of step d) can be added to the mixture of the particulate material and the at least one polysaccharide at a temperature of $\leq 100$ °C. For example, the at least one cross-linker of step c) and the optional at least one gel delaying agent of step d) can be added to the mixture of the particulate material and the at least one polysaccharide at a temperature of from 5 to 100 °C, preferably of from 5 to 80 °C, more preferably of from 10 to 60 °C and most preferably of from 15 to 40 °C, like of about room temperature.

The instant composition having a gel strength of from 1.0 $\mu$N to 10.0 N is preferably used in a method of hydraulically fracturing of a subterranean formation. Thus, according to a further aspect of the present invention, a method of hydraulically fracturing of a subterranean formation is provided, the method comprises at least the steps of:

a) providing a particulate material comprising at least one calcium carbonate having a weight median particle size $d_{50}$ value of 0.01 to 50.0 $\mu$m as defined above,
b) providing at least one polysaccharide comprising glucose units and/or galactose units and/or mannose units as defined above,
c) providing at least one cross-linker suitable for cross-linking the at least one polysaccharide of step b) as defined above,
d) providing a proppant having a Mohs hardness of at least 2.5,
e) mixing the particulate material of step a) with the at least one polysaccharide of step b) for forming a mixture of the particulate material and the at least one polysaccharide,
f) mixing the mixture obtained in step e) with the at least one cross-linker of step c) and the proppant of step d) for forming a hydraulic fracturing fluid, and
g) injecting the hydraulic fracturing fluid formed in step f) into a subterranean formation, at a flow rate from 150 to 300 1/s and a pressure rate from 500 to 1500 bar, for hydraulically fracturing the formation.

With regard to the particulate material provided in step a), the at least one polysaccharide provided in step b) and the at least one cross-linker of step c), it is referred to the definitions set out above when defining the instant composition and its single components.

The expression "hydraulically fracturing of a subterranean formation" refers to a method utilized for increasing the production of a well or for restoring the productivity of a non-productive well and is well known to the skilled person. This method typically includes injecting or pumping of a hydraulic fracturing fluid under high pressure down into a well bore and the adjacent subterranean formation, which results in an increased pressure in the adjacent subterranean formation to be treated. The increased pressure causes a fracturing of the subterranean formation resulting in an access for crude oil or gas to the well bore. The hydraulic fracturing fluid thus preferably comprises proppants for increasing the flow of the crude oil or gas through the fractures into the well bore.

Accordingly, a proppant having a Mohs hardness of at least 2.5 is provided in step d). In one embodiment of the present invention, the proppant has a Mohs hardness of at least 3.0. For example, the proppant has a Mohs hardness of from 2.5 to 9.0 or from 3.0 to 8.0.

The term "proppant" in the meaning of the present invention refers to any particulate solid material being stable at the conditions typically encountered during hydraulically fracturing of a subterranean formation. In particular, the proppant refers to a material being rigid and inert to crude oil, gas, water and fluids typically present in subterranean formations and/or compositions typically introduced for hydraulically fracturing of a subterranean formation.

It is thus appreciated that the proppant can be selected from any proppant typically used for hydraulically fracturing of a subterranean formation. For example, the proppant is selected from a material selected from the group comprising natural proppants, like mineral proppants, synthetic proppants, silica proppants, ceramic proppants, metallic proppants and mixtures thereof.

In one embodiment of the present invention, the proppant is selected from the group comprising sand, glass, ceramics, like lightweight ceramics, alumina, mica, corundum, bauxite, mullite, rutile, barite, silicon carbide, calcium oxide and mixtures thereof.

In one embodiment of the present invention, the proppant has a weight median particle size $d_{50}$ from 1.0 to 5 000.0 $\mu$m, preferably from 2.0 to 2 500.0 $\mu$m, more preferably from 3.0 to 1 000.0 $\mu$m, and most preferably from 4.0 to 500.0 $\mu$m.

The formation of the mixture of the particulate material and the at least one polysaccharide in step e) preferably takes place under mixing conditions. The skilled man will adapt these mixing conditions (such as the configuration of mixing pallets and mixing speed) according to his process equipment.

It is preferred that the particulate material comprising at least one calcium carbonate is provided in an amount of 1.0 to 60.0 wt.-%, based on the total weight of the mixture obtained in step e) or of the hydraulic fracturing fluid. For example, the particulate material is provided in an amount of from 1.0 to 50.0 wt.-%, preferably of from 5.0 to 40.0 wt.-% and most preferably of from 5.0 to 30.0 wt.-%, based on the total weight of the mixture obtained in step e) or of the hydraulic fracturing fluid.

It is appreciated that the particulate material comprising at least one calcium carbonate is provided in form of a powder or slurry. Preferably, the particulate material comprising at least one calcium carbonate is provided in form of a slurry.

If the particulate material is provided in form of a slurry, the slurry has solids content of at least 5.0 wt.-%, based on the total weight of the slurry. For example, the slurry has solids content of from 25.0 wt.-% to 85.0 wt.-%, more preferably from 35.0 wt.-% to 85.0 wt.-%, even more preferably from 40.0 wt.-% to 80.0 wt.-% and most preferably from 50.0 wt.-% to 80.0 wt.-%, based on the total weight of the slurry.

It is preferred that the slurry is an aqueous slurry.

It is a further requirement of the present invention that the at least one polysaccharide comprising glucose units and/or galactose units and/or mannose units is provided in an amount of 0.01 to 10.0 wt.-%, based on the total weight of the mixture obtained in step e) or of the hydraulic fracturing fluid. For example, the at least one polysaccharide is provided in an amount of 0.05 to 9.0 wt.-%, preferably of from 0.1 to 7.5 wt.-% and most preferably of from 0.5 to 5.0 wt.-%, based on the total weight of the mixture obtained in step e) or of the hydraulic fracturing fluid.

It is appreciated that the at least one polysaccharide comprising glucose units and/or galactose units and/or mannose units is provided in form of a hydrocolloidal suspension or a dry material. Preferably, the at least one polysaccharide comprising glucose units and/or galactose units and/or mannose units is provided in form of a hydrocolloidal suspension.

If the at least one polysaccharide comprising glucose units and/or galactose units and/or mannose units is provided in form of a hydrocolloidal suspension, the hydrocolloidal suspension has preferably a polysaccharide concentration from 0.01 to 4.0 wt.-%, based on the total weight of the hydrocolloidal suspension. For example, the hydrocolloidal suspension has a polysaccharide concentration from 0.01 to 3.5 wt.-%, more preferably from 0.05 to 3.0 wt.-% and most preferably from 0.1 to 2.5 wt.-%, based on the total weight of the suspension.

It is appreciated that the particulate material comprising at least one calcium carbonate and/or the at least one polysaccharide comprising glucose units and/or galactose units and/or mannose units is/are provided in combination with water. That is to say, the particulate material is provided in form of a slurry and/or the at least one polysaccharide is provided in form of a hydrocolloidal suspension.

Preferably, the particulate material is provided in form of a slurry and the at least one polysaccharide is provided in form of a hydrocolloidal suspension.

In one embodiment of the present invention, the particulate material is provided in form of a slurry and the at least one polysaccharide is provided in form of a dry material. Alternatively, the particulate material is provided in form of a powder and the at least one polysaccharide is provided in form of a hydrocolloidal suspension.

Accordingly, it is appreciated that the water present in the mixture obtained in step e) is preferably derived from the particulate material being provided in form of a slurry and/or the at least one polysaccharide being provided in form of a hydrocolloidal suspension.

In one embodiment of the present invention, mixing step e) is carried out in that the particulate material of step a) is added to the at least one polysaccharide of step b) to form a mixture of the particulate material and the at least one polysaccharide.

The step of mixing the particulate material of step a) with the at least one polysaccharide of step b) is preferably carried out in one or more steps.

For example, it is possible to add the particulate material of step a) to the at least one polysaccharide of step b) in one portion. In one embodiment of the present invention, the particulate material of step a) is added to the at least one polysaccharide of step b) in more than one portion, wherein said particulate material is preferably added in about equal portions. Alternatively, it is also possible to add the particulate material of step a) in unequal portions to the at least one polysaccharide of step b), i.e. in larger and smaller portions.

In one embodiment of the present invention, the particulate material of step a) can be added to the at least one polysaccharide of step b) in a broad temperature range. That is to say, the particulate material of step a) can be added to the at least one polysaccharide of step b) at a temperature of ≤ 100 °C. For example, the particulate material of step a) can be added to the at least one polysaccharide of step b) at a temperature of from 5 to 100 °C, preferably of from 5 to 80 °C, more preferably of from 10 to 60 °C and most preferably of from 15 to 40 °C, like of about room temperature. A temperature of ≤ 100 °C is preferably adjusted if the particulate material of step a) is provided as slurry and/or the at least one polysaccharide of step b) is provided as hydrocolloidal suspension.

The formation of the hydraulic fracturing fluid in step f) preferably takes place under mixing conditions. The skilled man will adapt these mixing conditions (such as the configuration of mixing pallets and mixing speed) according to his process equipment.

The step of mixing the mixture obtained in step e) with the at least one cross-linker of step c) and the proppant of step d) and is preferably carried out in one or more steps.

For example, it is possible to add the at least one cross-linker of step c) and the proppant of step d) to the mixture obtained in step e) in one portion. In one embodiment of the present invention, the at least one cross-linker of step c) and the proppant of step d) are added to the mixture obtained in step e) in more than one portion, wherein said at least one cross-linker and the proppant are preferably added in about equal portions. Alternatively, it is also possible to add the at least one cross-linker of step c) and the proppant of step d) in unequal portions to the mixture obtained in step e), i.e. in larger and smaller portions.

Preferably, the at least one cross-linker of step c) is added to the mixture obtained in step e) such that the hydraulic fracturing fluid comprises the at least one cross-linker in an amount of from 0.01 to 4.0 wt.-% wt.-%, preferably of from 0.01 to 3.5 wt.-%, more preferably of from 0.05 to 3.0 wt.-% and most preferably of from 0.1 to 2.5 wt.-%, based on the total weight of the hydraulic fracturing fluid.

Additionally or alternatively, the proppant of step d) is added to the mixture obtained in step e) such that the hydraulic fracturing fluid comprises the proppant in an amount of from 1.0 to 20.0 wt.-%, preferably from 2.0 to 15.0 wt.-%, based on the total weight of the hydraulic fracturing fluid.

In one embodiment of the present invention, the at least one cross-linker of step c) and the proppant of step d) can be added to the mixture obtained in step e) in a broad temperature range. That is to say, the at least one cross-linker of step c) and the proppant of step d) can be added to the mixture obtained in step e) in a temperature range of from -20 to 100 °C. For example, the at least one cross-linker of step c) and the proppant of step d) can be added to the mixture obtained in step e) in a temperature range of from 0 to 100 °C, preferably of from 5 to 90 °C, more preferably of from 10 to 80 °C and most preferably of from 15 to 70 °C. Preferably, said temperature ranges apply if the mixture obtained in step e) comprises solvent-free water.

Additionally or alternatively, the at least one cross-linker of step c) and the proppant of step d) can be added separately or together to the mixture obtained in step e).

In one embodiment of the present invention, the at least one cross-linker of step b) and the proppant of step d) are added together to the mixture obtained in step e). Preferably, the at least one cross-linker of step c) and the proppant of step d) are added separately to the mixture obtained in step e).

If the at least one cross-linker of step c) and the proppant of step d) are added separately to the mixture obtained in step e), the at least one cross-linker of step c) can be added before and/or after the proppant of step d) to the mixture obtained in step e). For example, the at least one cross-linker of step c) is added before the proppant of step d) to the mixture obtained in step e). Alternatively, the at least one cross-linker of step c) is added after the proppant of step d) to the mixture obtained in step e).

Preferably, the at least one cross-linker of step c) is added to the mixture obtained in step e) before the proppant of step d) is added to the mixture obtained in step e).

If the at least one cross-linker of step c) is added to the mixture obtained in step e) before the proppant of step d) is added to the mixture obtained in step e), it is appreciated that the composition having a gel strength of from 1.0 $\mu$N to 10.0 N as defined above is obtained.

In one embodiment of the present invention, mixing step f) further comprises adding at least one gel delaying agent in one or more steps to the mixture obtained in step e).

With regard to the at least one gel delaying agent optionally added in step e), it is referred to the definitions set out above when defining the optional at least one gel delaying agent in the instant composition having a gel strength of from 1.0 $\mu$N to 10.0 N.

In one embodiment of the present invention, the at least one gel delaying agent is added to the mixture obtained in step e) in one portion.

In another embodiment of the present invention, the at least one gel delaying agent is added to the mixture obtained in step e) in more than one portion, wherein said at least one gel delaying agent is preferably added in about equal portions. Alternatively, the at least one gel delaying agent is added to the mixture obtained in step e) in unequal portions, i.e. in larger and smaller portions.

If mixing step f) further comprises adding at least one gel delaying agent in one or more steps to the mixture obtained in step e), the at least one gel delaying agent is added in mixing step f) such that the hydraulic fracturing fluid comprises the at least one gel delaying agent in an amount of from 0.01 to 10.0 wt.-%, preferably of from 0.5 to 8.0 wt.-% and most preferably of from 1.0 to 6.0 wt.-%, based on the total weight of the hydraulic fracturing fluid.

The step of adding the at least one gel delaying agent to the mixture obtained in step e) preferably takes place under mixing conditions. The skilled man will adapt these mixing conditions (such as the configuration of mixing pallets and mixing speed) according to his process equipment.

If mixing step f) further comprises adding at least one gel delaying agent in one or more steps to the mixture obtained in step e), the at least one cross-linker of step c) and the at least one gel delaying agent can be added separately or together to the mixture obtained in step e).

In one embodiment of the present invention, the at least one cross-linker of step c) and the at least one gel delaying agent are added together to the mixture obtained in step e). In this case, the at least one cross-linker of step c) and the at least one gel delaying agent are preferably provided in form of a solution.

If the at least one cross-linker of step c) and the at least one gel delaying agent are added separately to the mixture obtained in step e), the at least one cross-linker of step c) can be added before and/or after the at least one gel delaying agent to the mixture obtained in step e). For example, the at least one cross-linker of step c) is added to the mixture obtained in step e) before the at least one gel delaying agent is added to the mixture obtained in step e).

It is appreciated that the mixing of the mixture obtained in step e) with the at least one cross-linker of step c) and the proppant of step d) forms the hydraulic fracturing fluid. In one embodiment of the present invention, the mixing of the mixture obtained in step e) with the at least one cross-linker of step c), the at least one gel delaying agent and the proppant of step d) forms the hydraulic fracturing fluid.

According to step g) of the instant process, the hydraulic fracturing fluid is injected into a subterranean formation, at a flow rate from 150 to 300 1/s and pressure rate from 500 to 1 500 bar, for hydraulically fracturing the formation.

In the method of the present invention, the hydraulic fracturing fluid can be injected into a subterranean formation by any conventional injecting means known to the skilled person. Typically, fracturing equipment operates over a range of pressures and injections rates such as from 500 to 1 500 bar, and preferably from 500 to 1 200 bar and 150 1/s to 300 1/s and preferably from 150 to 250 1/s. The skilled man thus will adapt the injecting conditions (such as the flow rate and the pressure rate) according to his process equipment and the subterranean formation to be treated.

It is appreciated that the subterranean formation to be treated can be any subterranean formation suitable for hydraulic fracturing. For example, the subterranean formation is an oil well or gas well.

In one embodiment of the present invention, the method further comprises step h) of contacting the hydraulic fracturing fluid in the subterranean formation with at least one gel breaking agent. The at least one gel breaking agent can be used for degrading the viscous hydraulic fracturing fluid and assists in the backflow of the hydraulic fracturing fluid once the hydraulic fracturing has been completed.

The hydraulic fracturing fluid can be contacted with any gel breaking agent which is suitable for degrading the cross-linked at least one polysaccharide.

In one embodiment of the present invention, the gel breaking agent is selected from enzymes, oxidants and mixtures thereof. For example, if the gel breaking agent is an oxidant, the gel breaking agent is selected from the group comprising ammonium perborate, sodium perborate, ammonium persulfate, sodium persulfate, calcium peroxide, magnesium oxide and mixtures thereof. Preferably, the gel breaking agent is selected from sodium perborate, ammonium persulfate and mixtures thereof.

Additionally or alternatively, the gel breaking agent can be an enzyme which is suitable for degrading the backbone of the at least one polysaccharide. For example, the gel breaking agent can be a hydrolase, preferably a hydrolase that is stable and remains active at a pH in the range of 6 to 12. Preferably, the hydrolase is selected from glycoside hydrolase, galactomannan hydrolase and mixtures thereof.

In one embodiment of the present invention, the amount of the gel breaking agent is selected such that the cross-linked at least one polysaccharide degrades in a desired time period. For example, the amount of the gel breaking agent is selected such that the cross-linked at least one polysaccharide degrades in a time period of from 1 min to 24 hours, preferably in a time period of from 30 minutes to 12 hours.

In one embodiment of the present invention, the amount of the gel breaking agent is from 0.01 to 50.0 wt.-%, preferably from 0.05 to 10.0 wt.-% and most preferably from 0.1 to 5.0 wt.-%, based on the total weight of the at least one polysaccharide in the hydraulic fracturing fluid.

In view of the very good results obtained, a further aspect of the present invention is directed to a hydraulic fracturing fluid comprising the instant composition. It is further required that the hydraulic fracturing fluid further comprises a proppant having a Mohs hardness of at least 2.5. For example, the proppant has a Mohs hardness of at least 3.0. Preferably, the proppant has a Mohs hardness of from 2.5 to 9.0 or from 3.0 to 8.0.

It is appreciated that the proppant can be selected from any proppant typically used for hydraulically fracturing of a subterranean formation. For example, the proppant is selected from a material selected from the group comprising natural proppants, like mineral proppants, synthetic proppants, silica proppants, ceramic proppants, metallic proppants and mixtures thereof.

In one embodiment of the present invention, the proppant is selected from the group comprising sand, glass, ceramics, like lightweight ceramics, alumina, mica, corundum, bauxite, mullite, rutile, barite, silicon carbide, calcium oxide and mixtures thereof.

In one embodiment of the present invention, the proppant has a weight median particle size $d_{50}$ from 1.0 to 5 000.0 $\mu$m, preferably from 2.0 to 2 500.0 $\mu$m, more preferably from 3.0 to 1 000.0 $\mu$m, and most preferably from 4.0 to 500.0 $\mu$m.

For example, the hydraulic fracturing fluid comprises the proppant in an amount of from 1.0 to 20.0 wt.-%, preferably from 2.0 to 15.0 wt.-%, based on the total weight of the hydraulic fracturing fluid.

In view of the very good results obtained with regard to the composition having a gel strength of from 1.0 $\mu$N to 10.0 N in hydraulically fracturing of a subterranean formation, as defined above, a further aspect of the present invention is directed to the use of the composition, as defined above, for hydraulic fracturing of a subterranean formation. It is preferred that the subterranean formation is an oil well or gas well. A still further aspect of the present invention is directed to the use of the composition, as defined above, for the preparation of a hydraulic fracturing fluid, comprising said composition and a proppant having a Mohs hardness of at least 2.5. The proppant is preferably selected from the group comprising sand, glass, ceramics, like lightweight ceramics, alumina, mica, corundum, bauxite, mullite, rutile, barite, calcium oxide and mixtures thereof. An even further aspect of the present invention is directed to the use of the composition, as defined above, in pharmaceutical applications, cosmetic applications, constructing applications, paper applications, paint applications, plastic applications, food applications and/or agricultural applications. With regard to the composition, it is referred to the definitions set out above when defining the composition and its components.

The following examples may additionally illustrate the invention but are not meant to restrict the invention to the exemplified embodiments. The examples below show the increased volatile onset temperature and the reduced moisture pick up susceptibility of the surface treated calcium carbonate-containing filler material according to the present invention:

EXAMPLES

Measurement methods

The following measurement methods are used to evaluate the parameters given in the examples and claims.

**Particle size distribution (mass % particles with a diameter < *X*) and weight median diameter ($d_{50}$) of a particulate material**

As used herein and as generally defined in the art, the "$d_{50}$" value is determined based on measurements made by using a Sedigraph™ 5100 of Micromeritics Instrument Corporation (operating instrument software version 1.04) and is defined as the size at which 50 % (the median point) of the particle volume or mass is accounted for by particles having a diameter equal to the specified value.

The method and the instrument are known to the skilled person and are commonly used to determine the particle size distribution of fillers and pigments. The measurement is carried out in an aqueous solution of 0.1 wt.-%

$Na_4P_2O_7$ and 0.05 wt.-% of a low molecular weight sodium polyacrylate dispersant. The samples are dispersed using a high speed stirrer and supersonics.

**BET specific surface area of a material**

Throughout the present document, the specific surface area (in $m^2/g$) of the particulate material is determined using the BET method (using nitrogen as adsorbing gas), which is well known to the skilled man (ISO 9277:1995). The total surface area (in $m^2$) of the mineral filler is then obtained by multiplication of the specific surface area and the mass (in g) of the mineral filler prior to treatment.

**Residual total moisture content measurement of calcium carbonate**

The residual total moisture content of the calcium carbonate is measured according to the Karl Fischer coulometric titration method, desorbing the moisture in an oven at 220°C and passing it continuously into the KF coulometer (Mettler Toledo coulometric KF Titrator C30, combined with Mettler oven DO 0337) using dry $N_2$ at 100 ml/min for 10 min. A calibration curve using water has to be made and a blind of 10 min gas flow without a sample has to be taken in account.

**Example 1**

The following components were used to prepare the composition of the instant invention:

Particulate material:

Norwegian marble rocks of the region of Molde, Norway, having a diameter of 10-300 mm were autogenously dry ground (i.e. in absence of grinding media) to a fineness of a $d_{50}$ in the range of 42.0 - 48.0 $\mu$m. Subsequently, the obtained dry ground material was wet ground at 10.0 - 15.0 wt.-% solid content in tap water in a vertical attritor mill (Dynomill) in a recirculation mode without adding additives, like dispersing and/or grinding aids. The wet grinding was carried out to a fineness until 60.0 wt.-% of the particles had a diameter < 1.0 $\mu$m and 8.0 wt.-% of the particles had a diameter < 0.2 $\mu$m. After wet grinding the obtained particulate material in the slurry had a median diameter $d_{50}$ of 0.8 $\mu$m and a specific surface area of 8.1 $m^2/g$. The obtained particulate material slurry was further thermally up-concentrated in an evaporator to a final particulate material content of 65.0 wt.-%, based on the total weight of the slurry, and further using 0.3 wt.-%, based on the total dry weight of the particulate material, of sodium polyacrylate having a molecular weight $M_w$ of 6 000 g/mol and 0.15 wt.-%, based on the total dry weight of the particulate material, of sodium hydrogen phosphate as dispersing agents.

Aqueous guar (hydrocolloidal) solution:

8.0 g guar (available from Sigma Aldrich) were added to 2 000 $cm^3$ of tap water under stirring. The hydrocolloidal suspension was stirred for 45 minutes at room temperature using a 70 mm dissolver disk at 1 500 rpm stirrer speed. The density of the hydrocolloidal suspension was 1.00 $g/cm^3$.

Cross-linker:

Solution of ammonium zirconium carbonate in water having an ammonium zirconium carbonate content of 30.0-% (available from Sigma Aldrich), and a density of 1.38 $g/cm^3$ at 25 °C.

**Test**

The following method was used to prepare the inventive composition as well as prior art compositions:

100 $cm^3$ of the aqueous guar (hydrocolloidal) suspension comprising the polysaccharide in an amount of 0.4 wt.-%, based on the total weight of the suspension, were placed in a 250 $cm^3$ glass beaker (diameter = 50 mm) and stirred with a magnetic stirrer with 500 - 1 000 rpm. Subsequently, water and/or particulate material slurry was/were added and the obtained mixture was stirred for 5 minutes. The cross-linker was then added and the obtained mixture was stirred for additional 3 seconds. Subsequently, the stirrer was stopped and the mixture was allowed to rest for 2 minutes.

The details regarding the amount of the guar suspension, water, particulate material slurry and cross-linker for the prior art compositions C1 to C6 as well as for inventive compositions I1 to I19 are summarized in Table 1.

Table 1: Details for the prior art compositions and the inventive compositions

| Test | Volume guar suspension [cm³] | Volume H$_2$O [cm³] | Mass GCC slurry [g] | Volume cross-linker [cm³] | Guar conc. [g/100cm³ H$_2$O] |
|------|------|------|------|------|------|
| C1 | 100 | 5.3 | 0 | 2 | 0.37 |
| C2 | 100 | 25 | 0 | 2 | 0.32 |
| C3 | 100 | 45 | 0 | 2 | 0.27 |
| C4 | 100 | 65 | 0 | 2 | 0.24 |
| C5 | 100 | 75 | 0 | 2 | 0.23 |
| C6 | 100 | 85 | 0 | 2 | 0.21 |
|  |  |  |  |  |  |
| I1 | 100 | 5.6 | 8 | 2 | 0.36 |
| I2 | 100 | 25 | 8 | 2 | 0.31 |
| I3 | 100 | 45 | 8 | 2 | 0.27 |
| I4 | 100 | 65 | 8 | 2 | 0.24 |
| I5 | 100 | 75 | 8 | 2 | 0.22 |
| I6 | 100 | 85 | 8 | 2 | 0.21 |
| I7 | 100 | 95 | 8 | 2 | 0.20 |
| I8 | 100 | 5.6 | 16 | 2 | 0.35 |
| I9 | 100 | 25 | 16 | 2 | 0.30 |
| I10 | 100 | 45 | 16 | 2 | 0.26 |
| I11 | 100 | 65 | 16 | 2 | 0.23 |
| I12 | 100 | 75 | 16 | 2 | 0.22 |
| I13 | 100 | 85 | 16 | 2 | 0.21 |
| I14 | 100 | 95 | 16 | 2 | 0.20 |
| I15 | 100 | 105 | 16 | 2 | 0.19 |
| I16 | 100 | 65 | 24 | 2 | 0.23 |
| I17 | 100 | 85 | 24 | 2 | 0.21 |
| I18 | 100 | 105 | 24 | 2 | 0.19 |
| I19 | 100 | 125 | 24 | 2 | 0.17 |

The gel strength of the prior art compositions C1 to C6 as well as of the inventive compositions I1 to I19 were determined by the following method:

Ceramic spheres having characteristics as outlined in Table 2 were placed on top of each prior art composition C1 to C6 as well as of inventive composition I1 to I19. Each test was started with sphere K7. If the sphere floated on the tested composition for 10 seconds, the sphere K7 was removed and the next sphere K6 was placed on the tested composition. If sphere K6 floated on the tested composition for 10 seconds, the sphere K6 was removed and the next sphere K5 was placed on the tested composition, and so on.

The test was stopped for each composition as soon as one of the respective tested spheres was completely covered with the tested composition, i.e. the sphere was not floating.

Table 2: Characteristics of the ceramic spheres

| Sphere | $m$ [g] | $d$ [mm] | $V$ [cm$^3$] | $\rho_p$ [gcm$^{-3}$] |
|---|---|---|---|---|
| K7 | 2.8 | 12.5 | 1.0 | 2.7 |
| K6 | 5.5 | 16.2 | 2.2 | 2.5 |
| K5 | 6.5 | 16.4 | 2.3 | 2.8 |
| K4 | 8.4 | 19.3 | 3.8 | 2.2 |
| K3 | 19.3 | 24.2 | 7.4 | 2.6 |
| K2 | 30.2 | 28.3 | 11.9 | 2.5 |
| K1 | 59.4 | 31.2 | 15.9 | 3.7 |

When determining the gel strength, it is assumed that the gel strength is equal to or larger than the drag force $F_D$ experienced by a sphere that floats on top of the respective composition. The drag force is given by the following Formula I:

$$F_D = (\rho_p - \rho_f)\frac{4}{3}\pi r^3 \qquad \text{I}$$

wherein

$\rho_p$ is the density of the respective sphere,
$\rho_f$ is the density of the tested composition, and
$r$ is the radius of the respective sphere.

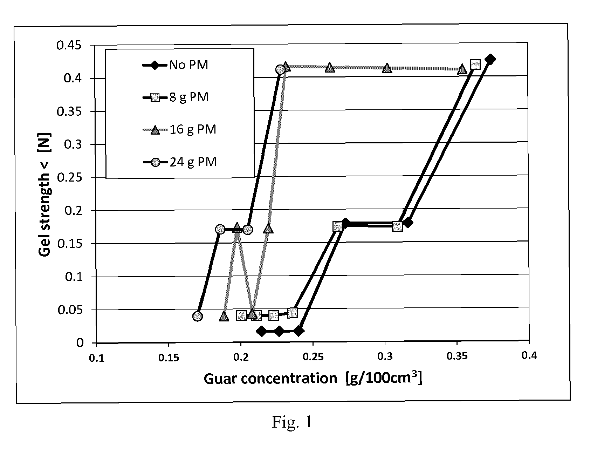

The gel strength determined for the prior art compositions C1 to C6 as well as for the inventive compositions I1 to I19 are outlined in the following Table 3 and Fig. 1:

Table 3: determination of gel strength

| | $\rho_f$ [g/cm$^3$] | Last sphere in test | Gel strength [N] |
|---|---|---|---|
| C1 | 1.011 | K1 | > 0.424 |
| C2 | 1.010 | K2 | > 0.178 |
| C3 | 1.008 | K2 | > 0.178 |
| C4 | 1.007 | - | < 0.017 |
| C5 | 1.007 | - | < 0.017 |
| C6 | 1.007 | - | < 0.017 |
| | | | |
| I1 | 1.058 | K1 | > 0.418 |
| I2 | 1.050 | K2 | > 0.174 |
| I3 | 1.043 | K2 | > 0.175 |
| I4 | 1.038 | K4 | > 0.044 |
| I5 | 1.036 | K5 | > 0.040 |
| I6 | 1.034 | K5 | > 0.040 |
| I7 | 1.032 | K5 | > 0.040 |
| I8 | 1.103 | K1 | > 0.411 |
| I9 | 1.088 | K1 | > 0.413 |
| I10 | 1.076 | K1 | > 0.415 |

(continued)

|  | $\rho_f$ [g/cm$^3$] | Last sphere in test | Gel strength [N] |
|---|---|---|---|
| I11 | 1.068 | K1 | > 0.416 |
| I12 | 1.064 | K2 | > 0.172 |
| I13 | 1.061 | K4 | > 0.043 |
| I14 | 1.058 | K2 | > 0.173[1] |
| I15 | 1.055 | K5 | > 0.040 |
| I16 | 1.096 | K1 | > 0.412 |
| I17 | 1.086 | K2 | > 0.170 |
| I18 | 1.078 | K2 | > 0.170 |
| I19 | 1.072 | K5 | > 0.039 |

From Table 3 and Fig. 1, it can be concluded that the inventive composition comprising a particulate material comprising at least one calcium carbonate having a weight median particle size $d_{50}$ value of 0.01 to 50.0 $\mu$m provides a sufficient rheology and, furthermore, provides the same or higher gel strength at a lower amount of a polysaccharide compared to the prior art compositions missing such particulate material.

**Claims**

1. A composition having a gel strength of from 1.0 $\mu$N to 10.0 N, the composition comprising:

   a) from 1.0 to 60.0 wt.-%, based on the total weight of the composition, of a particulate material comprising at least one calcium carbonate having a weight median particle size $d_{50}$ value of 0.01 to 50.0 $\mu$m;
   b) from 0.01 to 10.0 wt.-%, based on the total weight of the composition, of at least one polysaccharide comprising glucose units and/or galactose units and/or mannose units;
   c) from 0.01 to 4.0 wt.-%, based on the total weight of the composition, of at least one cross-linker suitable for cross-linking the at least one polysaccharide, and
   d) at least 26.0 wt.-%, based on the total weight of the composition, of water.

2. The composition of claim 1, wherein

   a) the at least one calcium carbonate of the particulate material is selected from among natural calcium carbonate (GCC), precipitated calcium carbonate (PCC), modified calcium carbonate (MCC) and mixtures thereof, and/or
   b) the particulate material further comprises a mineral material selected from mica, talc, dolomite, feldspar, bentonite, kaolinite, magnesite, muscovite, huntite and mixtures thereof.

3. The composition of claim 1 or 2, wherein the at least one calcium carbonate of the particulate material has

   a) a weight median particle size $d_{50}$ from 0.01 to 25.0 $\mu$m, preferably from 0.1 to 20.0 $\mu$m, more preferably from 0.1 to 10.0 $\mu$m, and most preferably from 0.2 to 5.0 $\mu$m, and/or
   b) a specific surface area of from 0.1 to 200.0 m$^2$/g, more preferably 3.0 to 25.0 m$^2$/g, even more preferably 5.0 to 15.0 m$^2$/g and most preferably 6.0 to 12.0 m$^2$/g, measured using nitrogen and the BET method.

4. The composition of any one of the preceding claims, wherein the at least one polysaccharide comprises

   a) glucose units, preferably a linear chain of 1,4-linked $\beta$-D-glucopyranosyl units, more preferably the linear chain of 1,4-linked $\beta$-D-glucopyranosyl units is 1,6-linked with $\alpha$-D-glucopyranosyl units, or
   b) galactose and mannose units, preferably a linear chain of 1,4-linked $\beta$-D-mannopyranosyl units, more preferably the linear chain of 1,4-linked $\beta$-D-mannopyranosyl units is 1,6-linked with $\alpha$-D-galactopyranosyl units.

5. The composition of claim 4, wherein the at least one polysaccharide comprising galactose and mannose units has a ratio of mannose units to galactose units from 6:1 1 to 1:1, preferably from 5:1 1 to 1:1, more preferably from 4:1 to 1:1 and most preferably from 3:1 to 1:1.

6. The composition of any one of the preceding claims, wherein

a) the at least one polysaccharide is cellulose and/or a cellulose derivative and/or guar and/or a guar derivative, and/or
b) the at least one polysaccharide is selected from the group comprising carboxymethyl cellulose, hydroxymethyl cellulose, hydroxyethyl cellulose, hydroxypropyl cellulose, hydroxyhexyl cellulose, guar gum, hydroxyethyl guar, hydroxypropyl guar, carboxymethyl guar, carboxymethyl hydroxyethyl guar, carboxymethyl hydroxypropyl guar, gum tragacanth, gum shiraz, gum ghatti, xanthan gum, scleroglucan gum, wellan gum, gelan gum and mixtures thereof.

7. The composition of any one of claims 1 to 3, wherein the at least one polysaccharide comprises galactose units and further units selected from arabinose units, rhamnose units, glucoronic acid units and mixtures thereof, preferably the at least one polysaccharide comprises galactose units, arabinose units, rhamnose units and glucoronic acid units.

8. The composition of any one of the preceding claims, wherein the at least one cross-linker is selected from the group comprising sodium tetraborate, a zirconium compound and mixtures thereof, preferably the at least one cross-linker is selected from the group comprising ammonium zirconium carbonate, potassium zirconium carbonate, zirconium lactate, zirconium glycolate, zirconium lactate triethanolamine, potassium pyroantimonate, potassium antimony tartrate, fused potassium antimony tartrate, antimony oxalate, antimony tartrate, and antimony ammonium fluoride and mixtures thereof.

9. The composition of any one of the preceding claims, wherein the composition further comprises at least one gel delaying agent, preferably a gel delaying agent selected from mono-, di- tri- or polyalcohols, alcohol amines, acidsand mixtures thereof.

10. The composition of any one of the preceding claims, wherein the composition has a pH in the range of 5 to 12, preferably in the range of 6 to 11.

11. A method of preparing a composition having a gel strength of from 0.01 to 10.0 N according to any one of claims 1 to 10, the method comprising at least the steps of:

a) providing a particulate material comprising at least one calcium carbonate as defined in any one of claims 1 to 3 in an amount of 1.0 to 60.0 wt.-%, based on the total weight of the composition;
b) providing at least one polysaccharide comprising glucose units and/or galactose units and/or mannose units as defined in any one of claims 1 or 4 to 7 in an amount of 0.01 to 10.0 wt.-%, based on the total weight of the composition;
c) providing at least one cross-linker as defined in any one of claims 1 or 8 in an amount of 0.01 to 4.0 wt.-%, based on the total weight of the composition;
d) optionally providing at least one gel delaying agent as defined in claim 9,
e) contacting the particulate material comprising at least one calcium carbonate of step a) with the at least one polysaccharide of step b), the at least one cross-linker of step c) and the optional at least one gel delaying agent of step d) to form the composition.

12. The method of claim 11, wherein

i) the particulate material comprising at least one calcium carbonate of step a) is provided in form of a powder or slurry, preferably a slurry having solids content of at least 5.0 wt.-%, preferably from 25.0 wt.-% to 85.0 wt.-%, more preferably from 35.0 wt.-% to 85.0 wt.-%, even more preferably from 40.0 wt.-% to 80.0 wt.-% and most preferably from 50.0 wt.-% to 80.0 wt.-%, based on the total weight of the slurry, and/or
ii) the at least one polysaccharide of step b) is in form of a hydrocolloidal suspension or a dry material, preferably in form of a hydrocolloidal suspension having a polysaccharide concentration from 0.01 to 4.0 wt.-%, preferably from 0.01 to 3.5 wt.-%, more preferably from 0.05 to 3.0 wt.-% and most preferably from 0.1 to 2.5 wt.-%, based on the total weight of the suspension, and/or
iii) the at least one cross-linker of step c) is in form of a solution or a dry material, preferably in form of a solution having a cross-linker concentration from 0.01 to 60.0 wt.-%, preferably from 1.0 to 50.0 wt.-%, more preferably

23

from 10.0 to 50.0 wt.-% and most preferably from 20.0 to 50.0 wt.-%, based on the total weight of the solution, and/or
iv) the optional at least one gel delaying agent of step d) is in form of a solution or a dry material, preferably in form of a dry material.

13. The method of any one of claims 11 or 12, wherein contacting step e) is carried out in that

   a) the particulate material of step a) is added to the at least one polysaccharide of step b) to form a mixture of the particulate material and the at least one polysaccharide, and
   b) the at least one cross-linker of step c) and the optional at least one gel delaying agent of step d) is/are added to the mixture of the particulate material and the at least one polysaccharide.

14. A method of hydraulically fracturing of a subterranean formation, preferably an oil well or gas well, the method comprising at least the steps of:

   a) providing a particulate material comprising at least one calcium carbonate having a weight median particle size $d_{50}$ value of 0.01 to 50.0 $\mu$m as defined in any one of claims 1 to 3,
   b) providing at least one polysaccharide comprising glucose units and/or galactose units and/or mannose units as defined in any one of claims 1 or 4 to 7,
   c) providing at least one cross-linker suitable for cross-linking the at least one polysaccharide of step b) as defined in any one of claims 1 or 8,
   d) providing a proppant having a Mohs hardness of at least 2.5,
   e) mixing the particulate material of step a) with the at least one polysaccharide of step b) for forming a mixture of the particulate material and the at least one polysaccharide,
   f) mixing the mixture obtained in step e) with the at least one cross-linker of step c) and the proppant of step d) for forming a hydraulic fracturing fluid, and
   g) injecting the hydraulic fracturing fluid formed in step f) into a subterranean formation, preferably an oil well or gas well, at a flow rate from 150 to 300 1/s and a pressure rate from 500 to 1 500 bar, for hydraulically fracturing the formation.

15. The method of claim 14, wherein the proppant is selected from the group comprising sand, glass, ceramics, like lightweight ceramics, alumina, mica, corundum, bauxite, mullite, rutile, barite, calcium oxide and mixtures thereof.

16. The method of claim 14 or 15, wherein the method further comprises step h) of contacting the hydraulic fracturing fluid in the subterranean formation with at least one gel breaking agent, preferably a gel breaking agent selected from enzymes, oxidants and mixtures thereof.

17. A hydraulic fracturing fluid comprising the composition as defined in any one of claims 1 to 10 and a proppant having a Mohs hardness of at least 2.5, preferably the proppant is selected from the group comprising natural proppants, like mineral proppants, synthetic proppants, silica proppants, ceramic proppants, metallic proppants and mixtures thereof.

18. Use of a composition according to any one of claims 1 to 10 for hydraulic fracturing of a subterranean formation, preferably an oil well or gas well.

19. Use of a composition according to any one of claims 1 to 10 for the preparation of a hydraulic fracturing fluid, comprising the composition according to any one of claims 1 to 10 and a proppant having a Mohs hardness of at least 2.5, preferably the proppant is selected from the group comprising sand, glass, ceramics, like lightweight ceramics, alumina, mica, corundum, bauxite, mullite, rutile, barite, calcium oxide and mixtures thereof.

20. Use of a composition according to any one of claims 1 to 10 in pharmaceutical applications, cosmetic applications, constructing applications, paper applications, paint applications, plastic applications, food applications and/or agri-cultural applications.

Fig. 1

Europäisches
Patentamt

European
Patent Office

Office européen
des brevets

# EUROPEAN SEARCH REPORT

Application Number

EP 13 19 6827

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/312968 A1 (LADVA HEMANT K J [US] ET AL) 28 November 2013 (2013-11-28) * paragraphs [0003], [0025], [0026]; examples 1,2,5 * * the whole document * | 1-20 | INV. C09K8/68 |
| A | US 2012/061083 A1 (BALLARD DAVID ANTONY [GB]) 15 March 2012 (2012-03-15) * examples 1-4 * * the whole document * | 1-20 | |
| A | US 4 620 596 A (MONDSHINE THOMAS C [US]) 4 November 1986 (1986-11-04) * column 6, lines 66-68; claim 1; tables 1,2 * | 1-20 | |

TECHNICAL FIELDS
SEARCHED       (IPC)

C09K

The present search report has been drawn up for all claims

| Place of search | Date of completion of the search | Examiner |
|---|---|---|
| The Hague | 9 May 2014 | Straub, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 19 6827

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2014

| Patent document cited in search report | | Publication date | Patent family member(s) | | Publication date |
|---|---|---|---|---|---|
| US 2013312968 | A1 | 28-11-2013 | NONE | | |
| US 2012061083 | A1 | 15-03-2012 | CA | 2754359 A1 | 26-08-2010 |
| | | | CN | 102365344 A | 29-02-2012 |
| | | | EA | 201171073 A1 | 30-03-2012 |
| | | | EP | 2398865 A1 | 28-12-2011 |
| | | | US | 2012061083 A1 | 15-03-2012 |
| | | | WO | 2010094937 A1 | 26-08-2010 |
| US 4620596 | A | 04-11-1986 | DE | 3378645 D1 | 12-01-1989 |
| | | | EP | 0159313 A1 | 30-10-1985 |
| | | | NO | 850448 A | 28-03-1985 |
| | | | US | 4620596 A | 04-11-1986 |
| | | | WO | 8501309 A1 | 28-03-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5874385 A
- GB 2382363 A
- US 1190112 A
- US 7135231 B1
- US 20030207768 A1
- US 20050211435 A1
- US 20090270282 A1
- US 20100300693 A1

**Non-patent literature cited in the description**

- **S. KESAVAN et al.** *Macromolecules,* 1992, vol. 25, 2026-2032
- **A. TAYAL et al.** *Macromolecules,* 1999, vol. 32, 5567-5574
- **R. BARATI et al.** *Journal of Applied Polymer Science,* 2012, vol. 126 (2), 587-592
- **H.R. JAFRY et al.** *Ind. Eng. Chem. Res.,* 2011, vol. 50 (6), 3259-3264
- **S. SHAH.** *SPE Production & Operations,* 2009, vol. 24 (3), 381-395
- **T.N. CASTRO DANTAS et al.** *Journal of Dispersion Science and Technology,* 2005, vol. 26 (1
- **M.M. SAMUEL et al.** *SPE Drilling & Completion,* 1999, vol. 14 (4), 240-246